(19) Europäisches Patentamt
European Patent Office
Office européen des brevets

(11) **EP 3 539 662 A1**

(12) **EUROPEAN PATENT APPLICATION**

(43) Date of publication:
**18.09.2019 Bulletin 2019/38**

(51) Int Cl.:
**B01L 3/00** (2006.01)      **C12N 5/00** (2006.01)
**B01F 13/00** (2006.01)      **B01L 7/00** (2006.01)
**B01F 3/08** (2006.01)      **C12M 1/32** (2006.01)
**C12M 3/06** (2006.01)      **C12Q 1/18** (2006.01)
**G01N 33/50** (2006.01)

(21) Application number: **19157685.9**

(22) Date of filing: **14.05.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2014   US 201461993119 P
13.02.2015   US 201562115877 P
13.02.2015   US 201562115872 P**

(62) Document number(s) of the earlier application(s) in accordance with Art. 76 EPC:
**15770965.0 / 3 142 790**

(71) Applicant: **Hooke Bio Limited
Clonlara, Co. Clare V94 EYK6 (IE)**

(72) Inventor: **DAVIES, Mark
Limerick (IE)**

(74) Representative: **Kirkham, Nicholas Andrew
Graham Watt & Co. LLP
St. Botolph's House
7-9 St. Botolph's Road
Sevenoaks, Kent TN13 3AJ (GB)**

Remarks:
This application was filed on 18-02-2019 as a divisional application to the application mentioned under INID code 62.

(54) **MICROFLUIDIC APPARATUS FOR FLUID HANDLING**

(57) The invention generally relates to microfluidic devices that include orthogonally positioned channels that are slidable relative to each other and methods of use thereof. In certain embodiments, the invention provides a microfluidic device that includes a first channel having an open end, and an open second channel. The first and second channels are slidable relative to each other such that when the open end of the first channel and the open portion of the open second channel are aligned with each other, fluid flows from the first channel into the second channel.

FIG. 13A

**Description**

Related Applications

This application claims the benefit of and priority to US provisional application number 61/993,119 filed May 14, 2014; US provisional application number 62/115,872 filed February 13,2015; and US provisional number 62/115,877 filed February 13, 2015, each of which is incorporated by reference.

Field of the Invention

The invention generally relates to microfluidic components that include open bottom wells within substrates and methods of use thereof.

Background

Drug discovery has a new twist: combining two old generic drugs to make a powerful new medicine, often for an entirely different disease. New and desirable combinations of old, off-patent drugs are hidden among millions of possible combinations. A new combination could shrink a tumor by combining an antipsychotic drug from the 1950s with a 1980s antibiotic; or a steroid could be combined with an anticoagulant that amplifies the steroid's desirable effects, thus lowering the dose and reducing side effects.

It is unknown which combination yields a therapeutic effect. Finding this perfect combination hinges on sheer luck and the brute-force of laboratory technicians. Only by testing each combination will a new therapeutic medicine be uncovered. Testing determines whether a drug pair inhibits the cells' production of inflammatory proteins, thus hinting that the combination might work against arthritis. Even pairing a small sample of over 2,000 generic drugs with one another and then testing each combination with cells overwhelms even the best stocked and well-staffed laboratories.

Testing two drugs in combination can yield millions of combinations; testing three drugs in combination can yield billions of combinations. Thus, the number of required Eppendorf tubes and sample well plates breaches laboratory capacities, frustrating the task of discovering new drug combinations.

Summary

The invention provides systems and methods for holding and manipulating, and combining fluids in drug screening assays. The invention includes substrates with open-bottom wells that can be loaded with fluids such as solutions of various compounds at various concentrations. When fluid is loaded into an open bottom well, the fluid is held within the well by surface tension. When another device, such as a channel, aligns with the open bottom well, the fluid flows from the open bottom well into the channel, which mechanism can be used to combine different fluids. When a device is not aligned with the open bottom well, fluid does not flow. Thus, the apparatuses of the invention are able to hold and manipulate fluids and--wittiout using pumps, valves, or electric fields--can be used to cross-combine many fluids thereby creating, for example, new combinations of existing drugs. Devices and methods of the invention accomplish this using gravity and surface tension to manipulate fluids and thus avoid the need for heat or electric fields which may interfere with the chemical combinations or cells being studied.

The devices and methods of the present invention allow for fluid containing living cells to be manipulated. Without pumps, valves or electric fields, cell integrity is persevered. Accordingly, systems and methods of the invention avoid the problems associated with using electrodes, pumps and other devices that lyse or rupture cells. The fluid-combining devices can further create cross-combinations of many compounds and expose the living cells to those novel combinations to probe the effects of those combinations on the cells. Hence, systems and methods of the invention are particularly amendable to a wide range of cellular assays and provide a mechanism by which new combinations of existing compounds may be created rapidly and even tested on living cells.

Using the systems and methods of the invention, drug screening assays within cells can be accomplished while maintaining the health and vitality of the cells. The open bottom wells allow for one, or a plurality of cells, to be held within each well. Fluids are introduced into each well, where the fluids can contain cell nutrients, dissolved gases, chemical species, or molecules. It should be appreciated that with devices of the invention, different types of cells (e.g. liver cells and lung cells) can be screened for possible drug interaction. Also, clusters of cells can be analyzed within the system of the invention.

The substrates of the invention can contain hundreds, thousands and tens of thousands of wells, allowing for tens of thousands of cells to be maintained and preserved during drug screening assays. This capacity accomplishes millions of cellular based drug assays in parallel.

In certain aspects, the invention provides a plurality of substrates comprising open bottom wells. In a simplistic view, a first substrate contains an open bottom well and a second substrate contains a channel. The first substrate and the second substrate are slidable relative to each other such that when the open bottom of the well is aligned with the channel, fluid flows from the open bottom well into the channel. The substrates may slide in any direction relative to each other, e.g., horizontally, vertically, diagonally, etc. Generally, systems of the invention are configured such that the open ends of the open bottom wells and the channels are exposed to atmospheric pressure.

In certain embodiments, the substrates are arranged in relation to each other such that an air gap exists when the open bottom well and the channel are aligned with each other. Fluid from the open bottom well bridges the air gap and enters the second channel. The open bottom well and channels are configured such that when they are not aligned, fluid does not flow there between.

In particular embodiments, gravitational force is used to produce and control flow within the system. In such embodiments, the substrates are arranged such that gravity causes flow of fluid within the open bottom well and the channel when aligned with each other.

While described in the context of an open bottom well and a channel for the sake of simplicity, the skilled artisan will recognize that the invention is not limited a single well and a single channel. The invention encompasses systems designed with a plurality of open bottom wells. When two substrates are aligned, a plurality of open bottom wells are aligned with channels, allowing fluid to flow there between.

Systems and methods of the invention can be loaded with any fluid(s) (e.g., liquid(s)). The fluid may be a single phase fluid. Fluids may contain reactants, elements, molecules, ions, cell nutrients, etc. Alternatively the fluid may include two phases, such as a fluid that includes droplets that are immiscible with the fluid. Such an exemplary two phase fluid is an oil that includes droplets of an aqueous fluid. In certain embodiments, the oil includes a surfactant. The droplets may include cells, nucleic acids, or reactants.

In some embodiments, the interior surface of the open bottom well is hydrophobic. In some embodiments, the interior surface of the open bottom well is hydrophilic. The open bottom well may be loaded with a hydrophilic or hydrophobic fluid.

Brief Description of the Drawings

FIG. 1 shows an embodiment of an open bottom well plate, or channel plate.

FIGS. 2A-2D show additional embodiments of the open bottom well plates, or channel plates.

FIG. 3 depicts several open bottom well plates or channel plates aligned.

FIGS. 4A-4B depict a multichannel system of the invention.

FIGS. 5A-5D depict alternate nonlinear forms of the channels or wells.

FIGS. 6A-6C show a multichannel system of the invention.

FIGS. 7A-7C show an embodiment of the invention.

FIGS. 8A-8C depict a multi-channel system.

FIGS. 9A-9B depict a multi-channel system.

FIGS. 10A-10E depict a multichannel system.

FIGS. 11A-11C depict alternate embodiments.

FIGS. 12A-12D show a multichannel system of the invention.

FIGS. 13A- 13B depict branched microfluidic channels.

FIGS. 14A-14E depict alternate embodiments.

FIG. 15 depicts an alternate embodiment.

FIG. 16 depicts an alternate embodiments of a multi-channel system.

FIGS. 17A-17B depict circular channel systems.

FIG. 18 depicts a multi-channel system.

FIG. 19 depicts a multi-channel system.

FIG. 20 depicts a multi-channel system.

FIG. 21 depicts a schematic of the channels and fluid flow.

FIG. 22 is a graph of dispensing time versus droplet volume.

FIGS. 23A and 23B depict substrates on a mechanical subsystem.

Detailed Description

The invention generally relates to open bottom well plates that are compatible with microfluidic devices and methods of use thereof.

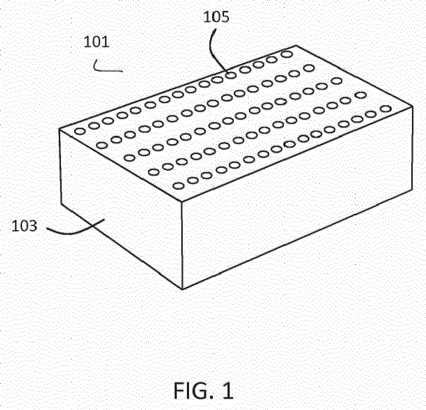

As shown in FIG. 1, an open bottom well plate 101 is shown with a plurality of wells 105. The open bottom wells may also be referred to as channels, and vice versa, herein. The open bottom wells are arranged vertically in a substrate **103.** The wells are open at both ends in the substrate (not shown). Fluid fills the wells, and the fluid is retained in the wells by surface tension. Several open bottom well plates may be used in a serial configuration, and may be aligned with other microfluidic components.

Channels

Substrates of the invention include channels that form the boundary for a fluid. A channel generally refers to a feature in a substrate that at least partially directs the flow of a fluid. In some cases, the channel may be formed, at least in part, by a single component, e.g., an etched substrate or molded unit The channel can have any cross-sectional shape, for example, circular, oval, triangular, irregular, square or rectangular (having any aspect ratio). The channel may be of any length and width to retain a fluid by surface tension.

A channel generally will include characteristics that facilitate control over fluid transport, e.g., structural characteristics (an elongated indentation) and/or physical or chemical characteristics (hydrophobicity vs. hydrophilicity) and/or other characteristics that can exert a force (e.g., a containing force) on a fluid. The fluid within the channel may partially or completely fill the channel. In some cases the fluid may be held or confined within the channel or a portion of the channel in some fashion, for example, using surface tension. In an article or substrate, some (or all) of the channels may be of a particular size or less, for example, having a largest dimension perpendicular to fluid flow of less than or equal to about 5 mm, less than or equal to about 2 mm, less than or equal to about 1 mm, less than or equal to about 500 microns, less than or equal to about 200 microns, less than or equal to about 100 microns, less than or equal to about 60 microns, less than or equal to about 50 microns, less than or equal to about 40 microns, less than or equal to about 30 microns, less than or equal to about 25 microns, less than or equal to about 10 microns, less than or equal to about 3 microns, less than or equal to about 1 micron, less than or equal to about 300 nm, less than or equal to about 100 nm, less than or equal to about 30 nm, or less than or equal to about 10 nm or less in some cases. Of course, in some cases, larger channels, tubes, etc. can be used to store fluids in bulk and/or deliver a fluid to the channel. In some embodiments, the channel is a capillary.

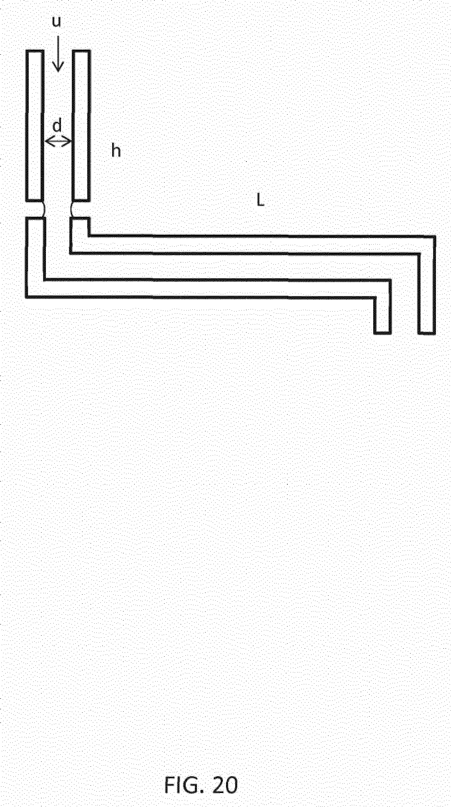

The channels of the invention are configured such that liquid is retained within the channel when it is completely out of alignment with another microfluidic channel (e.g., no overlap between open ends of channels). Liquid may be retained within the microfluidic channel due to surface tension. The flow in a microfluidic channel system, as shown in FIG. 20, with a height of h, an internal diameter of d, a length of L, a fluid, velocity of u, a fluid density of $\rho$, gravitation force of g, fluid viscosity of $\mu$, and surface tension of y, can be represented by the equation:

$$2\rho gh = \frac{\gamma\mu u(2h + L)}{(\frac{d}{2})^2}$$

or, rearranged as:

$$u = \frac{\rho ghd^2}{2\gamma\mu(2h + L)}$$

When fluid does not flow in the system, at maximum height, the equation becomes h = $\mu\gamma/d\rho$g.

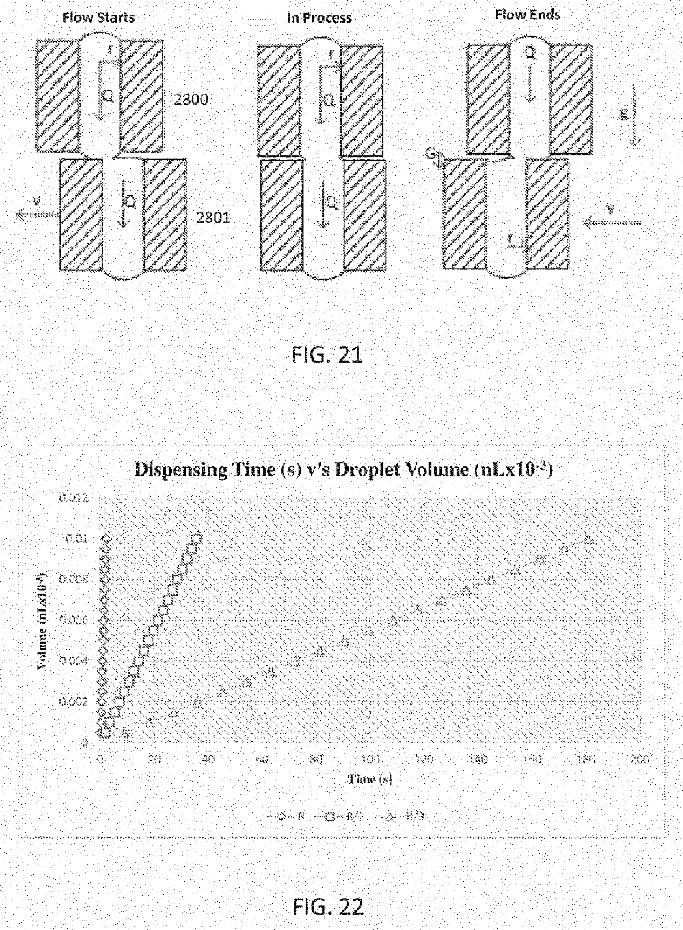

The volume of fluid that flows from one channel to another channel depends on the amount of time that the channels are aligned. As shown in FIG. 21, two channels 2800 and 2801 are aligned. Q is the flow rate in each channel, v is the velocity of the sliding channel, and r is the radius of the channel. Time when flowing is equal to nr/v, where n is the fraction of the lateral distance. As channel 2801 moves at a velocity relative to channel 2800, a volume of fluid flows from channel 2800 into channel 2801. G is the gap between the channels, and g is the force of gravity. The following equations denote the time required to dispense a volume, V from one channel to another channel. R is the resistance, P is the pressure, and u is the velocity.

$$R = \frac{8\mu L}{\pi r^4}, \qquad \Delta P = QR, \qquad \Delta P = \rho gh$$

$$\therefore \ \rho g h = Q\left(\frac{8\mu L}{\pi r^4}\right) = \pi r^2 u\left(\frac{8\mu L}{\pi r^4}\right)$$

$$\therefore \ \rho g h = u\left(\frac{8\mu L}{r^2}\right)$$

$Qt = V$ where V=volume dispensed.

$$\rho g h = \frac{V}{t}\left(\frac{8\mu L}{\pi r^4}\right)$$

For a given volume displaced we look to minimise time t.

$$t = \frac{V}{\rho g h}\left(\frac{2\mu L}{\pi r^4}\right)$$

$$\therefore \ t = \frac{8\mu L V}{\rho g h \pi r^4}$$

$h = L$ for vertical channels $\quad \therefore \ t = \frac{8\mu V}{\rho g \pi r^4} \quad$ This equation denotes the time required to dispense a volume, V.

FIG. 22 is a graph showing dispensing time (t) versus droplet volumes produced (nL) for varying vena contracta. The vena contracta means that average r is constantly changing, and can be averaged to r/2.

The dimensions of the channel may be chosen such that fluid is able to freely flow through the channel when channels are aligned and will not flow when channels are out of alignment with each other. The dimensions of the channel may also be chosen, for example, to allow a certain volumetric or linear flow rate of fluid in the channel. Of course, the number of channels and the shape of the channels can be varied by any method known to those of ordinary skill in the art. In some cases, more than one channel or capillary may be used. For example, two or more channels may be used, where they are positioned inside each other, positioned adjacent to each other, etc.

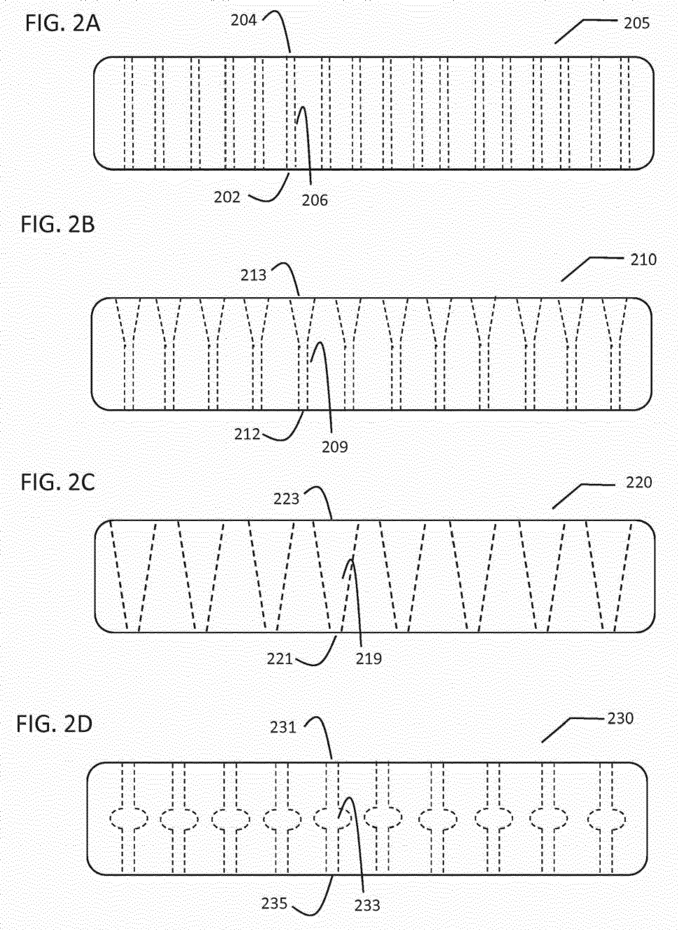

The channels of the device of the present invention can be of any geometry as described. It should be appreciated that the channels can also be referred to as open bottom wells. Hence channel may be used interchangeably with open bottom well. However, the channels or open bottom wells of the device can comprise a specific geometry such that the contents of the channel or open bottom well are manipulated, e.g., sorted, mixed, prevent clogging, etc. For example, for channels/open bottom wells that are configured to carry droplets, the channels of the device may preferably be square, with a diameter between about 2 microns and 1 mm. This geometry facilitates an orderly flow of droplets in the channels. FIGS. 2A-2D depict possible channel geometries, or open bottom well geometries. As shown in FIG. 2A, the channel plate **205** with the channels **206** are composed of walls that are parallel, when viewed from the vertical cross-sectional plane. The channels **206** have open ends **202** and **204.** Open ends **202** and **204** may be at atmospheric pressure. As shown in FIG. 2B, the channel plate **210** has channels **209** that have walls that form a funnel shape. The channels **206** have open ends **213** and **212.** FIGS. 2C and 2D show alternate channel designs in channel plates **220** and **230.** FIG. 2C shows channels **219** that have walls that are slanted, and FIG. 2D shows channels **233** that have a geometry which includes a small reservoir. FIG. 2C also depicts that the channels are opened ended at **223** and **221.** The channels **233** in FIG. 2D are open ended at **235** and **231.**

To prevent material (e.g., cells and other particles or molecules) from adhering to the sides of the channels, the channels may have a coating which minimizes adhesion. Such a coating may be intrinsic to the material from which the device is manufactured, or it may be applied after the structural aspects of the channels have been microfabricated. The walls of the channels may be hydrophobic or hydrophilic. TEFLON (polymer, commercially available from DuPont, Inc.), or polytetrafluoroethylene (PTFE), is an example of a coating that has suitable surface properties. A substrate containing open microfluidic channels may be constructed from PTFE, or PTFE containing materials. Microfluidic channels may be constructed from PTFE, or PTFE containing materials. Additionally, or in the alternative, microfluidic channels may be coated with PTFE. In a preferred embodiment of the invention, the walls of the interior portion of the microfluidic channels are composed of PTFE, or a material containing PTFE, to render the interiors of the microfluidic channels hydrophobic.

The surface of the channels can be coated with any anti-wetting or blocking agent for the dispersed phase. The channel can be coated with any protein to prevent adhesion of the biological/chemical sample. For example, in one embodiment the channels are coated with BSA, PEG-silane and/or fluorosilane. For example, 5 mg/mL BSA is sufficient to prevent attachment and prevent clogging. In $\alpha$-tiother embodiment, the channels can be coated with a cyclized transparent optical polymer obtained by copolymerization of perfluoro (alkenyl vinyl ethers), such as the type sold by Asahi Glass Co. under the trademark Cytop. In such an embodiment, the coating is applied from a 0.1-0.5 wt % solution of Cytop CTL-809M in CT-Solv 180. This solution can be injected into the channels of a microfluidic device via a plastic syringe. The device can then be heated to about 90°C for 2 hours, followed by heating at 200°C for an additional 2 hours. In another embodiment, the channels can be coated with a hydrophobic coating of the type sold by PPG Industries, Inc. under the trademark Aquapel (e.g., perfluoroalkylalkylsilane surface treatment of plastic and coated plastic substrate surfaces in conjunction with the use of a silica primer layer) and disclosed in U.S. Pat. No. 5,523,162, which patent is hereby incorporated by reference. By fluorinating the surfaces of the channels, the continuous phase preferentially wets the channels and allows for the stable generation and movement of droplets through the device.

The surface of the channels can be also fluorinated to prevent undesired wetting behaviors. For example, a microfluidic device can be placed in a polycarbonate dessicator with an open bottle of (tridecafluoro-1,1,2,2-tetrahydrooctyl) trichlorosilane. The dessicator is evacuated for 5 minutes, and then sealed for 20-40 minutes. The dessicator is then backfilled with air and removed. This approach uses a simple diffusion mechanism to enable facile infiltration of channels of the microfluidic device with the fluorosilane and can be readily scaled up for simultaneous device fluorination.

The substrates may be formed by known methods in the art. The substrates may be formed by several different types of materials, such as silicon, plastic, quartz, glass, plastic, or other suitable materials. Also, it should be appreciated that the size, shape and complexity of the microfluidic channels and structures that can be used in the microfluidic device depends on the materials used and the fabrication processes available for those materials. Typical system fabrication includes making trenches in a conducting material (silicon) or in a non-conducting substrate (e.g., glass or plastic) and converting them to channels by bonding a cover plate to the substrate. See for example, U.S. Patent 6,210,986. In addition, for example, U.S. Patent No. 5,885,470 teaches a microfluidic device having application in chemistry, biotechnology, and molecular biology that provides precise control of fluids by forming various grooves or channels and chambers in a polymeric substrate. The process of forming microfluidic channels in a substrate can include wet chemical etching, photolithographic techniques, controlled vapor deposition, and laser drilling into a substrate.

Alternative techniques for constructing channels may be employed in the fabrication of the devices of the invention. For example, in M. Stjemstrom and J. Roeraade, Method for Fabrication of Microfluidic Systems in Glass, J. Micromechanics and Microenginneering, 8, 33-38, 1998, walls are formed that define the channels rather than simply forming trenches in the substrate. The silicon nitride channels and are formed by conformal coating etched features in a silicon wafer with deposited silicon nitride. The silicon nitride channels are bonded to the glass substrate by an intermediate thermal oxide layer grown on the surface of the silicon nitride. The silicon wafer is etched away leaving silicon nitride channels on the surface of the glass substrate. An electrically insulating material can be applied to the substrate to support the silicon nitride structures.

Channel plates of the present invention may be formed by injection molding. In this process of preparing a microfluidic device by injection molding, in some methods, a polymeric material is injected into an injection molding mold or mold insert and the polymeric material is cured in the model to form the substrate of the microfluidic device. However, an injection molding mold or mold insert may be prepared from materials such as metal, silicon, ceramic, glass, quartz, sapphire and polymeric materials. The forming of the negative impression of the channel architecture may be achieved by techniques such as photolithographic etching, stereolithographic etching, chemical etching, reactive ion etching, laser machining, rapid prototyping, ink-jet printing and electroformation. With electroformation, the injection molding mold or mold insert is formed as the negative impression of the channel architecture by electroforming metal, and the metal mold is polished, preferably polished to a mirror finish. The devices of the present invention may be manufactured by injection molding using any suitable thermoplastic, for example, polycyclic olefin polyethylene co-polymers, poly methyl methacrylate (PMMA), polycarbonate, polyalkanes and polystyrenes. The microfluidic devices can be fabricated in accordance with the invention by compression molding and casting on a wide range of polymers. Polymers preferred for microfluidic devices are low melt viscosity polymers with minimal amount of leachable additives, for example, polycyclic olefin polyethylene co-polymers.

Ink-jet technology may be applied in fabricating the channel plates directly, or in fabricating the molds used in making the channel plates by injection molding. Ink-jet printing technology provides the desired microfluidic features to be printed directly on a substrate such as glass, ceramics, silicon, polymers or any organic, inorganic or hybrid materials that form a flat surface for the printing of features. A negative of the microfluidic features may be made by conventional electroplating with copper or nickel, or any other metals over the device made via printing technology. The materials forming the microfluidic features may be organic, inorganic, or a blend of organic and inorganic materials. See for example, WIPO Patent Application WO/2002/063288. 3D printing or additive manufacturing may be applied to fabricate microfluidic devices of the invention. This additive process creates an object by laying down successive layers of material until the entire object is created. Selective laser sintering (SLS) and fused deposition modeling (FDM) are the most common technologies using this way of printing. Another method of printing is to lay liquid materials that are cured with different technologies. The most common technology using this method is called stereolithography (SLA).

Channels may be formed by the sealing of numerous layers. For example, the deposition of a thin film on one of two glass substrates followed by an anodic (also frequently called electrostatic) bonding process. This metallic or semiconducting layer can be used as an intermediate layer. An example of this method is described in the article "Glass-to-glass anodic bonding with standard ICAechnology thin films as intermediate layers," by A. Berthold et. al., Sensors & Actuators A Vol. 82,2000, pp. 224-228. Additionally, anodic bonding of a glass to a silicon substrate is a method that can be employed in the fabrication of the device of the invention. See for example US Patent Number 3,397,278. Bonding between two insulator substrates can be accomplished with direct anodic bonding. See for example U.S. Patent Number US 3,506,424. This method comprises the evaporation of a thin layer of SiO on thin film circuitry, present on a substrate, and subsequent anodic bonding of a glass foil. Glass layers may be bonded by thermal glass-to-glass bonding, which consists in heating both substrates to a temperature at which melting starts to occur, or at least to a temperature at which the glass starts to soften, e.g. at 550 degrees C, and pressing the substrates together, by which a bond is formed. Bonding of two glass substrates through an intermediate layer of a low-melting-point material, or through an intermediate layer which solidifies from a solution during heat treatment is summarized in H.Y. Wang et al., "Low temperature bonding for microfabrication of chemical analysis systems," Sensors & Act. B vol. 45, 1997, p. 199-207. It should be appreciated that known techniques can be employed for scaling or joining layers of materials in forming microfluidic devices of the invention.

Channel plates for analysis or synthesis of biological and chemical species can be fabricated from two flat electrically insulating glass substrates, with one substrate containing an etched microfluidic channel and drilled or etched access-holes. The glass plates are bonded together so that the microfluidic channel in one substrate forms together with the second glass substrate a microfluidic channel. Ample illustrative examples of such devices can be found in literature, DJ. Harrison and co-workers, in: "Capillary electrophoresis and sample injection systems integrated on a planar glass chip," Analytical Chemistry vol. 64, 1 Sept. 1992, p. 1926, which describes a micromachined glass chip, which employs electrokinetic and electroosmotic principles for sample preparation and liquid propulsion, and demonstrates electrophoresis on the chip.

Various layers can be formed to define the walls of the channels. For example, the substrate may comprise various glass layers, and may include an elastomeric layer, wherein two glass layers interfaced to form one or more microfluidic channels. An elastomeric layer may be positioned between glass layers to form one or more microfluidic channels. For example, layers may include borosilicate glasses, pyrex, borofloat glass, Corning 1737, Corning Eagle 2000, silicon acrylic, polycarbonate, liquid crystal polymer, polymethylmethoxyacrylate (PMMA), Zeonor, polyolefin, polystyrene, polypropylene, and polythiols. Depending on the choice of the material different fabrication techniques may also be used.

Channel plates of the invention may be made out of plastic, such as polystyrene, using a hot embossing technique. In that process, the microfluidic channels are embossed into the plastic to create the bottom surface. A top layer may then be bonded to the bottom layer. In an alternative fabrication method, the use of epoxy casting techniques to create the microfluidic channels through the use of UV or temperature curable epoxy on a master that has the negative replica of the intended structure can be employed. Laser or other types of micromachining approaches may also be utilized to create microfluidic channels.

Channel plates of the invention can be manufactured to represent any geometry, and to include any number of channels. Substrates may be manufactured to have 6, 24, 96, 384 or even 1536 channels. Substrates may be manufactured to have 3456 or even 9600 channels. The substrates may be manufactured to have any array or arrangement of channels. For example channels may be arranged in a 2:3 rectangular matrix.

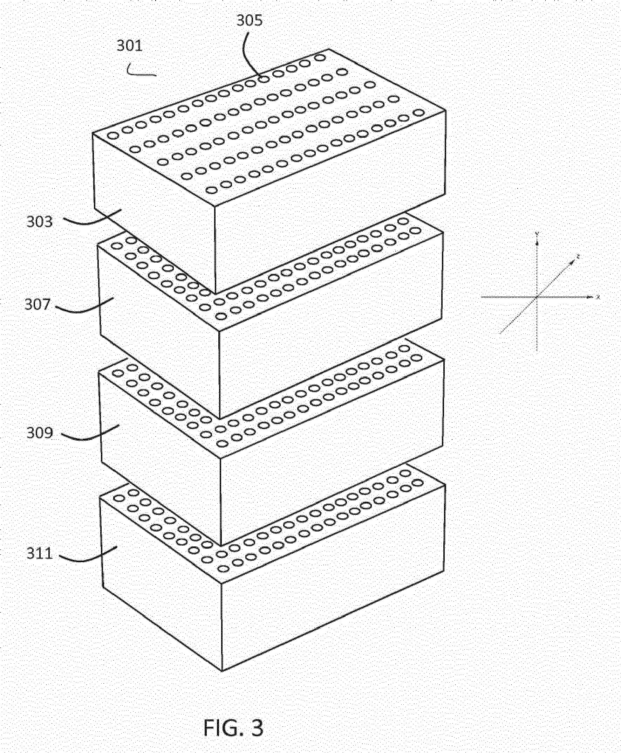

Channel plates of the invention may be aligned in a serial configuration. As shown in FIG. 3, an arrangement of channel plates **301** comprises channel plates **303, 307, 309,** and **311**. The channel plates contain channels **305** that are opened at both ends. Channel plates **303**, **307, 309,** and **311** can be arranged so that fluid flows there between. Also, channel plates **303, 307, 309,** and **311** can be arranged so that fluid is retained within the channels of the plates and does not flow into the channels of the other plates. For example, channel plate **303** can be arranged or aligned with channel plate **307** so that fluid flows from the channels in channel plate **303** into the channels of channel plate **307.** The channel plates of the invention may be moved in any direction in the x-y-z plane. Additionally, the channel plates may move independently of other channel plates.

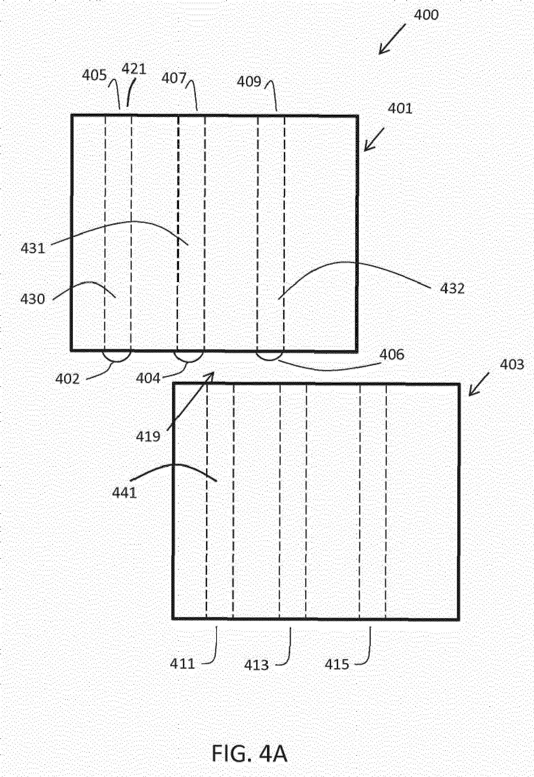

The channel plates of the invention can be moved in any direction in the x-y-z plane, referred to herein as sliding, to align with another channel plate or other microfluidic component. When aligned, channel plates of the invention allow fluid to flow from the channels of one plate into the channels of another plate. Additionally, the channel plates can be aligned so that all channels align, or the channel plates can be aligned so that only a fraction of the number of channels align. FIGS. 4A- 4B show another exemplary embodiment of a system **400** of the invention in which the channels are formed in multiple substrates **401** and **403**. As shown in FIGS. 4A-4B, the microfluidic channels are housed within two different substrates that move relative to one another. The substrates may move in any direction within the x-y-z plane. Either substrate **401** or **403** may be stationary, in which only one of the substrates moves relative to the other. In other embodiments, both substrates **401** and **403** may be slidable or moveable. As discussed above, the substrates may be formed from any materials and by any techniques discussed above. Substrate **401** contains microfluidic channels **405, 407,** and **409,** and substrate **403** contains microfluidic channels **411, 413** and **415**. In addition, the channels have ends that are open. In this specific embodiment, the microfluidic channels are open ended to atmospheric pressure. For example, microfluidic channel **405** is open to the atmosphere at **402** and **421**.

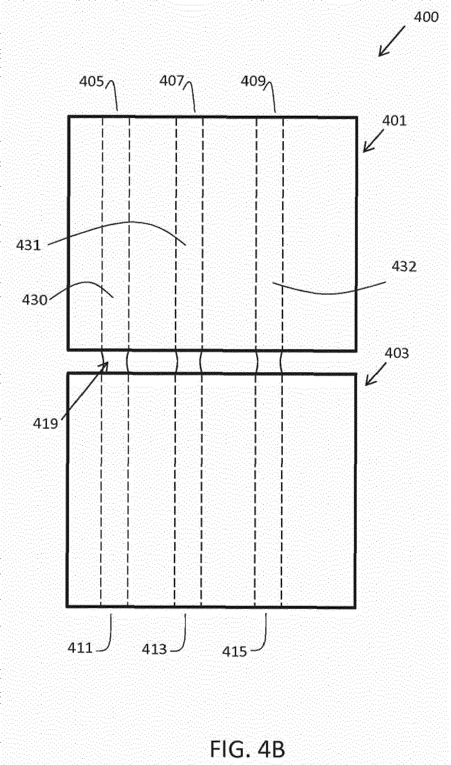

As shown in FIG. 4A, substrates **401** and **403** are positioned so that the microfluidic channels are not aligned. However, as discussed above, the substrates are moveable or slidable and therefore can be positioned so that microfluidic channels align. The substrates can be moved in any direction relative to each other, such as vertically or horizontally, or any direction in three dimensional space. FIG. 4A shows substrates **401** and **403** in which the microfluidic channels are not aligned. In that position, fluid within microfluidic channels **405, 407,** and **409** is not able to flow. For example, fluid **430** housed in microfluidic channel **405** is open to atmospheric pressure, however, due to channel dimensions and forces such as surface tension, fluid **430** remains in microfluidic channel **405**. Fluids **430, 431,** and **432** may be the same or different. Substrate **401** and/or **403** can slide to align the microfluidic channels. Either substrate **401** or **403** can slide, or both substrates **401** or **403** can slide to align the microfluidic channels. As shown in FIG. 4B, substrates **401** and **403** are positioned so that the microfluidic channels are aligned. The substrates do not need to align so that the surfaces of the substrate are flush. The substrates may be aligned to create an air gap **419**. As shown in FIG. 4B, alignment of the microfluidic channels allows fluid to bridge the air gap **419** and flow from substrate **401** to substrate **403**. For example, as shown in FIG. 2B, microfluidic channel **405** is aligned with microfluidic channel **411** thereby creating an arrangement in which fluid bridges the air gap **419** and flows between microfluidic channels **405** and **411.**

In a preferred embodiment, referring to FIGS. 4A and 4B, substrate **401** is initially not aligned with substrate **403**. Substrate **403** is then positioned so one channel in substrate **403** aligns with at least one channel in substrate **401**. Substrate **403** is then positioned out of alignment with substrate **403**. Substrate **403** is then aligned so the channel in substrate **403** aligns with a different channel in substrate **401.** This process may be repeated to distribute the fluids in substrate **401** with the fluids housed in substrate **403.**

Alignment includes complete alignment, partial alignment, and misalignment. In complete alignment, the menisci of the two microfluidic channels are in intact and the center axes of the microfluidic channels are in alignment. In partial alignment, the center axes are not aligned, however, there is partial overlap of the first and second channels such that the distance between the center axes is sufficiently small so that flow between the two microfluidic channels occurs. In complete misalignment, there is no overlap between the channels and the distance between the center axes is sufficiently great so that flow between the two microfluidic channels does not occur. In the present invention, alignment is meant to encompass both complete and partial alignment. The device of the invention flows fluid between two microfluidic channels even in the cases of partial alignment.

In certain embodiments, the ends of the channels are open to atmospheric pressure. As shown in FIGS. 4A and 4B, the open ends **405, 407, 409, 402, 404**, and **406** are exposed to atmospheric pressure. In such embodiments, as depicted in FIG. 4B, the channel **405** and channel **411** may be arranged in relation to each other such that an air gap **419** exists between the channels. As shown in FIG. 4B, when channel **405** and the channel **411** are aligned with each other, fluid **430** from channel **405** bridges the air gap **419** and enters channel **411**.

In an aspect of the invention, the air gap may comprise any known gas, at any temperature and pressure. The air gap may be at atmospheric pressure and be comprised of air. However, the air gap is not limited to atmospheric pressure or air. In some embodiments, the devices of the present invention may be completely or partially enclosed within a chamber and the chamber may be filled with a gas other than air. The pressure can be above or below atmospheric pressure and the temperature can be at, above, or below room temperature, which is about 37 degrees Celsius. Systems of the invention can be equipped with any type of flow driving mechanism, such as pumps.

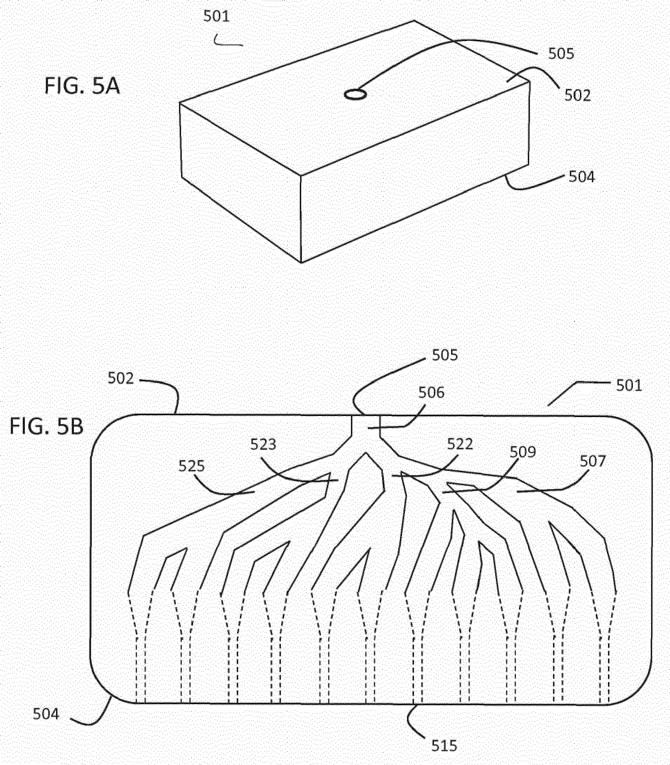

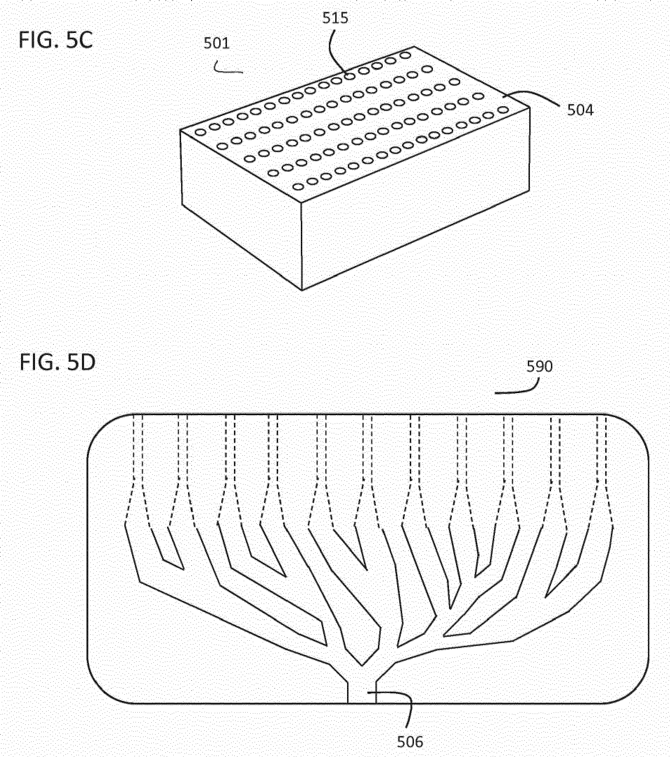

Channels within channel plates of the invention may be linear, or nonlinear. Channels within channel plates may be curved, or have other nonlinear configurations. In addition, a channel cross sectional diameter may remain constant, or the diameter may widen or narrow. Channels within channel plates may be branched, containing one or multiple branches within the channel. For example, FIGS. 5A-5D depict a channel plate with branched channels. As shown in FIG. 5A, channel plate **501** has a top portion **502** and a bottom portion **504**. The top portion **502** contains the open end **505** of a channel. FIG. 5B depicts a cross-sectional view of channel plate **501**, showing the open end **505** of channel **506**. Channel **506** branches into numerous channels, for example channel **507**, **509, 522, 523,** and **525**. It should be appreciated that the channels can have numerous branches as needed. These channels further branch into a plurality of channels, with open ends **515.** FIG. 5C depicts channel plate **501,** showing surface **504** with a plurality of open ends **515** of channels. It should be appreciated that a fluid can be flowed into channel **506** to fill the branched channels. FIG. 5D depicts a channel plate in the opposite configuration, where a plurality of channels can feed fluid to channel **506.**

Multichannel and multicomponent systems

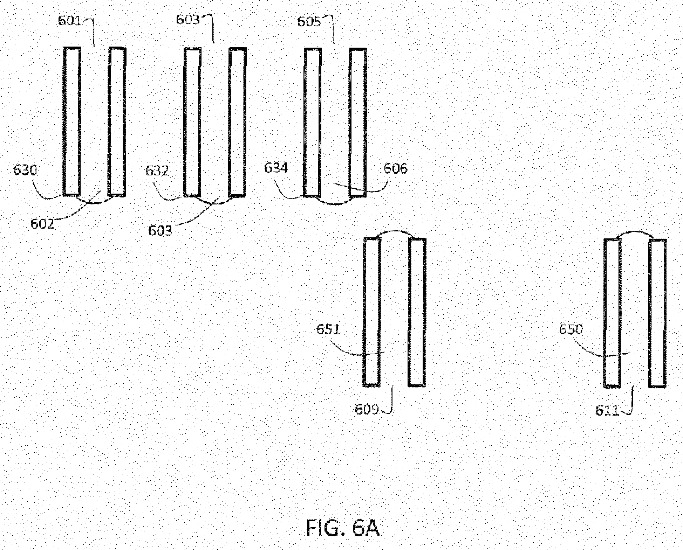

In certain embodiments, the systems of the invention are multichannel and multicomponent systems. There is no limit to the number of channels that can be included in systems of the invention, nor is there any limitation on the configuration of the channels. A multi-channel system is described in FIG. 6A, and the skilled artisan will appreciate that this is only a single example of a multi-channel system. Numerous other multichannel and multicomponent systems and configurations can be envisioned by the skilled artisan and are within the scope of the invention.

FIGS. 6A-6C depict a multi-channel system configured to allow microfluidic channels to slide or move relative to each other to alter alignment of the channels. FIG. 6A shows multiple microfluidic channels **601, 603,** and **605** which are open at ends **630, 632,** and **634.** Microfluidic channels **601, 603,** and **605** each may contain a fluid, for example microfluidic channel **601** contains fluid **602.** Fluids **602, 603,** and **606** may be the same or different. Each fluid **602, 603,** and **606** is retained in the microfluidic channels due to channel geometry and by forces such as surface tension. As discussed above, an aspect of the invention is that fluid does not flow from the microfluidic channel unless aligned with another microfluidic channel. Additionally, microfluidic channels **601**, **603,** and **605** may be slidable or moveable together or independent of one another. FIG. 6A also depicts microfluidic channels **609** and **611** which are open ended at **640** and **641.** Microfluidic channels **609** and **611** are shown in FIG. 6A to contain fluids **650** and **651.** However, microfluidic channels **609** and **611** are not required to contain fluids and may not contain fluids. Microfluidic channels **609** and **611** may be moved independent of one another or may be moved together. As shown in FIG. 6A, microfluidic channels **609** and **611** are positioned to be disengaged from microfluidic channels **601, 603,** and **605.** In this positioning of the microfluidic channels, microfluidic channels **601**, **603,** and **605** are prevented from flowing fluid due to the physical properties of the microfluidic channel and the fluid, e.g. surface tension.

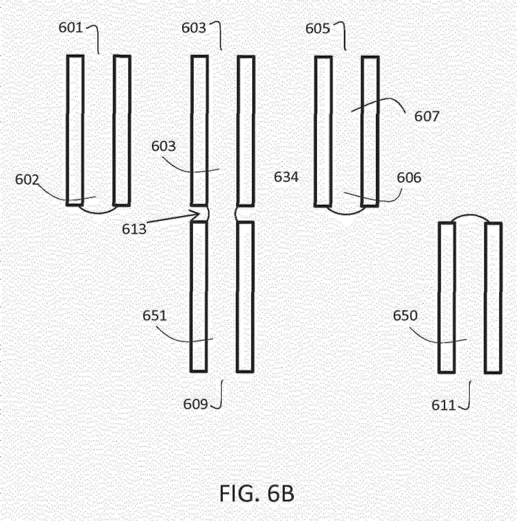

Microfluidic channels **609** and **611** maybe slid or moved to align with any of the microfluidic channels **601, 603,** or **605.** FIG. 6B depicts microfluidic channels **609** and **611** that has been moved or slid relative to microfluidic channels **601, 603,** or **605.** As shown in FIG. 6B, microfluidic channel **609** has been slid to engage at least one of microfluidic channels **601, 603,** and **605.** Moving or sliding of microfluidic channel **609** or **611** may involve movement in any plane or direction. In FIG. 6B, microfluidic channel **603** is aligned with microfluidic channel **609.** The alignment may cause an air gap **613.** Also, as discussed above, it is not necessary for the microfluidic channels to be aligned so that the microfluidic channels are flush. Rather, an air gap **613** may be present between the two microfluidic channels. The arrangement of microfluidic channels **603** and **609** is such when the channels are aligned, fluid bridges the air gap **613** and flows from microfluidic channel **603** into microfluidic channel **609.** In this positioning, microfluidic channel **609** receives fluid **603** from microfluidic channel **603.**

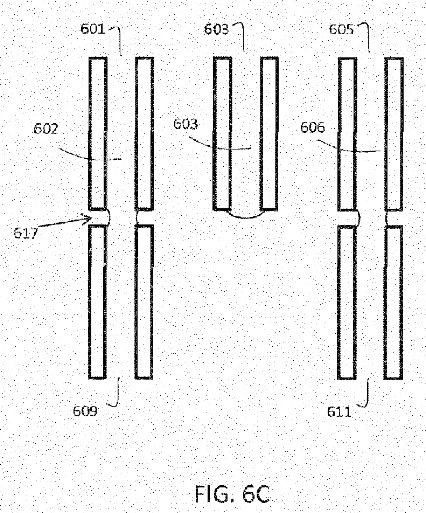

The microfluidic channels of the invention may be slid or move in several iterations. For example, as shown in FIG. 6C, microfluidic channel **609** has been slid to align with microfluidic channel **601.** As discussed previously, microfluidic channel **609** was aligned with microfluidic channel **603** and received fluid **603.** Microfluidic channel **609** now contains fluid **603** and fluid **602.** In this embodiment, fluids are mixed from two different microfluidic channels. Additionally, as shown in FIG. 6C, microfluidic channel **611** is aligned with microfluidic channel **605.** It should be appreciated that microfluidic channels **609** and **611** could be slid or moved at the same time, or independently of each other, depending on the configuration of microfluidic channels **609** and **611** and their respective substrates.

It should be appreciated that the multichannel systems of the invention may include numerous channels aligned in various planes of space. For example, FIGS. 6A-6C serve to illustrate how two levels of microfluidic channels can align to direct the flow of droplets within a microfluidic system. It should be appreciated that numerous levels of microfluidic channels may include a multichannel system, as discussed below.

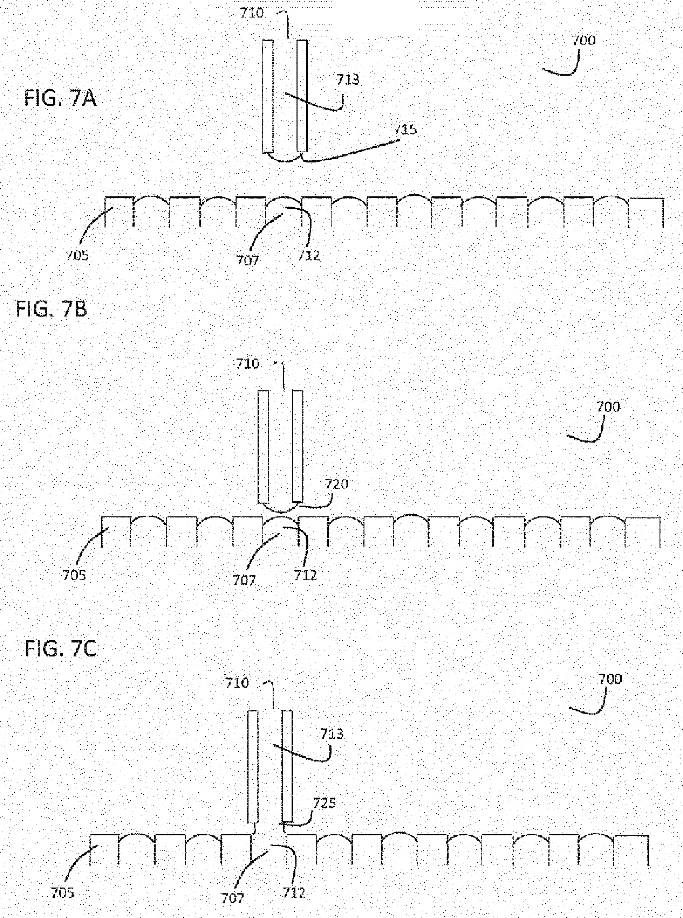

FIGS. 7A - 7C demonstrate the transfer of fluid from a microfluidic channel to a channel plate. FIGS. 7A-7C depict a microfluidic system **700.** Microfluidic system **700** is shown with a microfluidic channel **710** and a channel plate **705** that contains a plurality of microfluidic channels **707.** Microfluidic channels **707** contain a fluid **712.** Microfluidic channel **710** contains a fluid **713** and has an open end **715.** As shown in FIG. 7A, the fluid **713** is contained within microfluidic channel **710.** When microfluidic channel **710** is not aligned with another microfluidic channel, the fluid is retained due to channel geometry and surface tension. As shown in FIG. 7B, microfluidic channel **710** is positioned to align with microfluidic channel **707** in channel plate **705.** Microfluidic channels **710** and **707** are aligned so that the center axes of the microfluidic channels are substantially aligned. Microfluidic channels **710** and **707** are also aligned to create an air gap **720.** As shown in FIG. 7C, the fluids **713** and **712** combine at air gap **720,** allowing fluid **713** to flow into microfluidic channel **707.** The flow can be due to gravitational forces, or other forces acted on the system **700.**

It should be appreciated that the microfluidic channels of the invention can be arranged in any configuration.

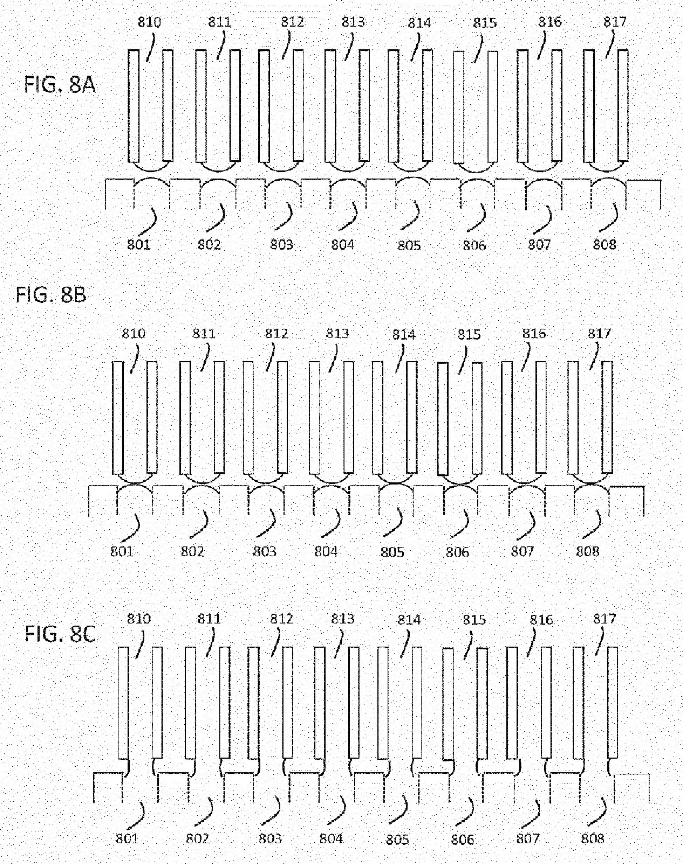

Similar to the arrangement depicted in FIGS. 7A - 7C, FIGS. 8A - 8C depict a microfluidic system **800** in which microfluidic channels **810** - **817** are configured to align with a series of microfluidic channels **801-806** in channel plate **890**. Any number of microfluidic channels can be arranged. The series of microfluidic channels contain fluids. As shown in FIG. 8A, when the microfluidic channels are not aligned, fluid does not flow. As shown in FIG. 8B, microfluidic channels **810** - **817** are positioned to align with at least one of the microfluidic channels **801-806** in channel plate **890**. When a microfluidic channel **801-806** is aligned with a microfluidic channel **810** - **817** so that the center axes of the microfluidic channels are substantially aligned, and the menisci are in contact, fluid flows from the microfluidic channel into the microfluidic channel in the channel plate. It should be appreciated that one or more microfluidic channels may align. Also, it should be appreciated, that the series of microfluidic channels **810** - **817** or the series of microfluidic channels **801-806** in channel plate **890** can be slid to align at least one or more microfluidic channels, disengage alignment, and then align with at least one other microfluidic channels.

Fluids and Droplets

As discussed above, the microfluidic systems of the present invention are capable of controlling the direction and flow of fluids and entities within the device. The term flow generally refers to any movement of liquid or solid through a device or in a method of the invention, and encompasses without limitation any fluid stream, and any material moving with, within or against the stream, whether or not the material is carried by the stream. The application of any force may be used to provide a flow, including without limitation, pressure, capillary action, electro-osmosis, electrophoresis, dielectrophoresis, optical tweezers, gravity, and combinations thereof, without regard for any particular theory or mechanism of action, so long as molecules, cells or virions are directed for detection, measurement or sorting according to the invention. Specific flow forces are described in further detail herein.

A droplet, as used herein, is an isolated portion of a first fluid that substantially or completely surrounded by a second fluid. In some cases, the droplets may be spherical or substantially spherical; however, in other cases, the droplets may be non-spherical, for example, the droplets may have the appearance of blobs or other irregular shapes, for instance, depending on the external environment. As used herein, a first entity is surrounded by a second entity if a closed loop can be drawn or idealized around the first entity through only the second entity. The dispersed phase fluid can include a biological/chemical material. The biological/chemical material can be tissues, cells, particles, proteins, antibodies, amino acids, nucleotides, small molecules, and pharmaceuticals. The biological/chemical material can include one or more labels known in the art. The label can be a DNA tag, dyes or quantum dot, or combinations thereof

The term emulsion generally refers to a preparation of one liquid distributed in small globules (also referred to herein as drops or droplets) in the body of a second liquid. The first and second fluids are immiscible with each other. For example, the discontinuous phase can be an aqueous solution and the continuous phase can be a hydrophobic fluid such as an oil. This is termed a water in oil emulsion. Alternatively, the emulsion may be an oil in water emulsion. In that example, the first liquid, which is dispersed in globules, is referred to as the discontinuous phase, whereas the second liquid is referred to as the continuous phase or the dispersion medium. The continuous phase can be an aqueous solution and the discontinuous phase is a hydrophobic fluid, such as an oil (e.g., decane, tetradecane, silicon, or hexadecane). The droplets or globules of oil in an oil in water emulsion are also referred to herein as "micelles", whereas globules of water in a water in oil emulsion may be referred to as "reverse micelles".

The fluidic droplets may each be substantially the same shape and/or size. Alternatively, a first type droplet may be considerably larger than a second type droplet. The shape and/or size can be determined, for example, by measuring the average diameter or other characteristic dimension of the droplets. The average diameter of a plurality or series of droplets is the arithmetic average of the average diameters of each of the droplets. Those of ordinary skill in the art will be able to determine the average diameter (or other characteristic dimension) of a plurality or series of droplets, for example, using laser light scattering, microscopic examination, or other known techniques. The diameter of a droplet, in a non-spherical droplet, is the mathematically-defined average diameter of the droplet, integrated across the entire surface. The average diameter of a droplet (and/or of a plurality or series of droplets) may be, for example, less than about 1 mm, less than about 500 micrometers, less than about 200 micrometers, less than about 100 micrometers, less than about 75 micrometers, less than about 50 micrometers, less than about 25 micrometers, less than about 10 micrometers, or less than about 5 micrometers in some cases. The average diameter may also be at least about 1 micrometer, at least about 2 micrometers, at least about 3 micrometers, at least about 5 micrometers, at least about 10 micrometers, at least about 15 micrometers, or at least about 20 micrometers in certain cases. Droplets may vary in size, where a first type droplet is larger than a second type droplet.

The droplet forming liquid is typically an aqueous buffer solution, such as ultrapure water (e.g., 18 mega-ohm resistivity, obtained, for example by column chromatography), 10 mM Tris HCl and 1 mM EDTA (TE) buffer, phosphate buffer saline (PBS) or acetate buffer. Any liquid or buffer that is physiologically compatible, with the population of molecules, cells or particles to be analyzed and/or sorted can be used. The fluid passing through the main channel and in which the droplets are formed is one that is immiscible with the droplet forming fluid. The fluid passing through the main channel can be a non-polar solvent, decane (eg., tetradecane or hexadecane), fluorocarbon oil, silicone oil or another oil (for example, mineral oil).

The dispersed phase fluid may also contain biological/chemical material (e.g., molecules, cells, or other particles) for combination, analysis and/or sorting in the device. The droplets of the dispersed phase fluid can contain more than one particle or can contain no more than one particle. For example, where the biological material comprises cells, each droplet preferably contains, on average, no more than one cell. However, in some embodiments, each droplet may contain, on average, at least 1000 cells. The droplets can be detected and/or sorted according to their contents.

The concentration (i.e., number) of molecules, cells or particles in a droplet can influence sorting efficiently and therefore is preferably optimized. In particular, the sample concentration should be dilute enough that most of the droplets contain no more than a single molecule, cell or particle, with only a small statistical chance that a droplet will contain two or more molecules, cells or particles. This is to ensure that for the large majority of measurements, the level of reporter measured in each droplet as it passes through the detection module corresponds to a single molecule, cell or particle and not to two or more molecules, cells or particles.

The parameters which govern this relationship are the volume of the droplets and the concentration of molecules, cells or particles in the sample solution. The probability that a droplet will contain two or more molecules, cells or particles ($P_{\leqq2}$) can be expressed as

$$P_{\leqq2}=1-\{1+[\text{cell}]\times V\}\times e^{-[\text{cell}]\times V}$$

where "[cell]" is the concentration of molecules, cells or particles in units of number of molecules, cells or particles per cubic micron ($\mu m^3$), and V is the volume of the droplet in units of $\mu m^3$

It will be appreciated that $P_{\leqq2}$ can be minimized by decreasing the concentration of molecules, cells or particles in the sample solution. However, decreasing the concentration of molecules, cells or particles in the sample solution also results in an increased volume of solution processed through the device and can result in longer run times. Accordingly, it is desirable to minimize to presence of multiple molecules, cells or particles in the droplets (thereby increasing the accuracy of the sorting) and to reduce the volume of sample, thereby permitting a sorted sample in a reasonable time in a reasonable volume containing an acceptable concentration of molecules, cells or particles.

The maximum tolerable $P_{\leqq2}$ depends on the desired purity of the sorted sample. The purity in this case refers to the fraction of sorted molecules, cells or particles that possess a desired characteristic (e.g., display a particular antigen, are in a specified size range or are a particular type of molecule, cell or particle). The purity of the sorted sample is inversely proportional to $P_{\leqq2}$. For example, in applications where high purity is not needed or desired a relatively high $P_{\leqq2}$ (e.g., $P_{\leqq2}=0.2$) may be acceptable. For most applications, maintaining $P_{\leqq2}$ at or below about 0.1, preferably at or below about 0.01, provides satisfactory results.

The fluidic droplets may contain additional entities, for example, other chemical, biochemical, or biological entities (e.g., dissolved or suspended in the fluid), cells, particles, gases, molecules, or the like. In some cases, the droplets may each be substantially the same shape or size, as discussed above. In certain instances, the invention provides for the production of droplets consisting essentially of a substantially uniform number of entities of a species therein (i.e., molecules, cells, particles, etc.). For example, about 90%, about 93%, about 95%, about 97%, about 98%, or about 99%, or more of a plurality or series of droplets may each contain the same number of entities of a particular species. For instance, a substantial number of fluidic droplets produced, e.g., as described above, may each contain 1 entity, 2 entities, 3 entities, 4 entities, 5 entities, 7 entities, 10 entities, 15 entities, 20 entities, 25 entities, 30 entities, 40 entities, 50 entities, 60 entities, 70 entities, 80 entities, 90 entities, 100 entities, etc., where the entities are molecules or macromolecules, cells, particles, etc. In some cases, the droplets may each independently contain a range of entities, for example, less than 20 entities, less than 15 entities, less than 10 entities, less than 7 entities, less than 5 entities, or less than 3 entities in some cases. In some embodiments, a droplet may contain 100,000,000 entities. In other embodiments, a droplet may contain 1,000,000 entities.

In a liquid containing droplets of fluid, some of which contain a species of interest and some of which do not contain the species of interest, the droplets of fluid may be screened or sorted for those droplets of fluid containing the species as further described below (e.g., using fluorescence or other techniques such as those described above), and in some cases, the droplets may be screened or sorted for those droplets of fluid containing a particular number or range of entities of the species of interest, e.g., as previously described. Thus, in some cases, a plurality or series of fluidic droplets, some of which contain the species and some of which do not, may be enriched (or depleted) in the ratio of droplets that do contain the species, for example, by a factor of at least about 2, at least about 3, at least about 5, at least about 10, at least about 15, at least about 20, at least about 50, at least about 100, at least about 125, at least about 150, at least about 200, at least about 250, at least about 500, at least about 750, at least about 1000, at least about 2000, or at least about 5000 or more in some cases. In other cases, the enrichment (or depiction) may be in a ratio of at least about $10^4$, at least about $10^5$, at least about $10^6$, at least about $10^7$, at least about $10^8$, at least about $10^9$, at least about $10^{10}$, at least about $10^{11}$, at least about $10^{12}$, at least about $10^{13}$, at least about $10^{14}$, at least about $10^{15}$, or more. For example, a fluidic droplet containing a particular species may be selected from a library of fluidic droplets containing various species, where the library may have about 100, about $10^3$, about $10^4$, about $10^5$, about $10^6$, about $10^7$, about $10^8$, about $10^9$, about $10^{10}$, about $10^{11}$, about $10^{12}$, about $10^{13}$, about $10^{14}$, about $10^{15}$, or more items, for example, a DNA library, an RNA library, a protein library, a combinatorial chemistry library, etc. In certain embodiments, the droplets carrying the species may then be fused, reacted, or otherwise used or processed, etc., as further described below, for example, to initiate or determine a reaction.

In some aspects of the invention the droplets may comprise sample fluid, discussed below. It should be appreciated that the sample fluid varies depending on the biological or chemical assay being performed within the droplet. In assays involving biological processes, the sample fluid is typically an aqueous buffer solution, such as ultrapure water (e.g., 18 mega-ohm resistivity, obtained, for example by column chromatography), 10 mM Tris HCl and 1 mM EDTA (TE) buffer, phosphate buffer saline (PBS) or acetate buffer. In assays involving amplification and detection of nucleic acids, any liquid or buffer that is physiologically compatible with nucleic acid molecules can be used.

In assays related to cell culturing, the sample fluid may comprise cell medium. The cell medium provides the necessary nutrients, growth factors, and hormones for cell growths, as well as regulating the pH and the osmotic pressure of the culture. The cell culture medium may allow for and support growth of the cells thus being cultured, or the cell medium is a maintenance medium. Growth is understood as an increase in viable cell density during at least a certain period of the cell culture. A maintenance medium is a cell culture medium which supports cell viability but which does not encourage cell growth. See for example, cell culture medium related patents: US Patent 4,038,139, 1977; US Patent 7,258,998, 2007; US Patent App. 13/497,707,2010; US Patent 8,338,177, 2012; and US Patent App. 13/695,002, 2011. The growth medium controls the pH of the culture and buffers the cells in culture against fluctuations in the pH. This buffering may be achieved by including an organic (e.g., HEPES) or $CO_2$ bicarbonate based buffer. Control of pH is needed to ensure the growth and health of cells in culture. Most normal mammalian cell lines grow well at pH 7.4, and there is very little variability among different cell strains.

The carrier fluid is one that is immiscible with the sample fluid. As used herein, carrier fluid and immisible fluid may be used interchangeably. The carrier fluid can be a non-polar solvent, decane (eg., tetradecane or hexadecane), fluorocarbon oil, silicone oil or another oil (for example, mineral oil). In an aspect of the invention, the carrier fluid forms a meniscus at the open end of the microfluidic channel, caused by surface tension. The meniscus can be either convex or concave, depending on the carrier fluid and the surface of the microfluidic channel.

In certain embodiments, the carrier fluid contains one or more additives, such as agents which increase, reduce, or otherwise create non-Newtonian surface tensions (surfactants) and/or stabilize droplets against spontaneous coalescence on contact. Surfactants can include Tween, Span, fluorosurfactants, and other agents that are soluble in oil relative to water. In some applications, performance is improved by adding a second surfactant, or other agent, such as a polymer or other additive, to the sample fluid. Surfactants can aid in controlling or optimizing droplet size, flow and uniformity. Furthermore, the surfactant can serve to stabilize aqueous emulsions in fluorinated oils from coalescing.

In certain embodiments, the droplets may be coated with a surfactant or a mixture of surfactants. Preferred surfactants that may be added to the carrier fluid include, but are not limited to, surfactants such as sorbitan-based carboxylic acid esters (e.g., the "Span" surfactants, Fluka Chemika), including sorbitan monolaurate (Span 20), sorbitan monopalmitate (Span 40), sorbitan monostearate (Span 60) and sorbitan monooleate (Span 80), and perfluorinated polyethers (e.g., DuPont Krytox 157 FSL, FSM, and/or FSH). Other non-limiting examples of non-ionic surfactants which may be used include polyoxyethylenated alkylphenols (for example, nonyl-, p-dodecyl-, and dinonylphenols), polyoxyethylenated straight chain alcohols, polyoxyethylenated polyoxypropylene glycols, polyoxyethylenated mercaptans, long chain carboxylic acid esters (for example, glyceryl and polyglycerl esters of natural fatty acids, propylene glycol, sorbitol, polyoxyethylenated sorbitol esters, polyoxyethylene glycol esters, etc.) and alkanolamines (e.g., diethanolamine-fatty acid condensates and isopropanolamine-fatty acid condensates).

Droplet Formation by Fluid Segmentation within Movable Channels

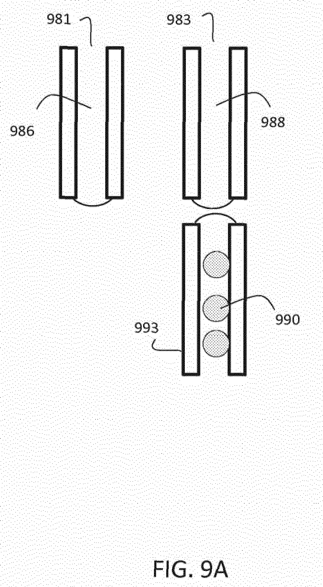

Microfluidic systems of the invention can be used to form droplets through the movement of the different channels. The microfluidic device can be used to produce single or multiple emulsions with precise control of both the contents and size of the drops. FIG. 9A depicts an arrangement of microfluidic channels for forming droplets. Microfluidic channel 981 contains a fluid 986 which is immiscible with aqueous fluids, such as an oil. Microfluidic channel 983 contains an aqueous fluid 988. To create droplets within microfluidic channel 993, microfluidic channels aligns with microfluidic channel 981 to form an air gap (not shown) where the fluid 986 spans the air gap and flows into microfluidic channel 993. As discussed herein, microfluidic channels are aligned by sliding one microfluidic channel proximate to another microfluidic channel, causing an air gap between the microfluidic channels to form. Microfluidic channel 993 is disengaged or misaligned with microfluidic channel 981 to allow a small volume of fluid 986 to flow into microfluidic channel 993.

Microfluidic channel **993** is then aligned with microfluidic channel **983** to allow a small volume of aqueous fluid **988** to flow into microfluidic channel **993**. Fluid **988** is immiscible with fluid **986** present in microfluidic channel **993** causing droplets **990** to form, which contains fluid **988**. Microfluidic channel **993** can align in an alternating pattern to form droplets **990**. In the alternative, oil droplets can be formed in an aqueous phase using the technique described above.

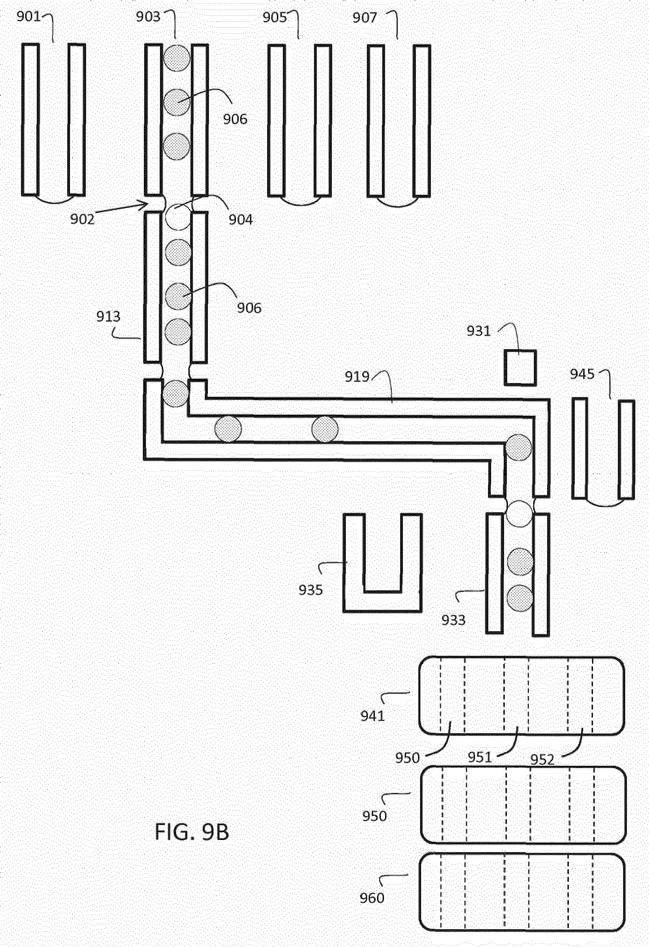

FIG. 9B depicts an arrangement using microfluidic systems of the invention to form droplets from the fluid contained in microfluidic channel **901** and insert the formed droplets into a stream of droplets from microfluidic channel **903**. As shown in FIG. 9B, microfluidic channel **913** can align with several microfluidic channels: **901, 903, 905,** and **907**. In aspect of the invention, microfluidic channel **913** can be slid or moved in order to align with microfluidic channels **901, 903, 905,** and **907**. Microfluidic channels **901, 903, 905,** and **907** may be stationary or moveable. Furthermore, microfluidic channels **901, 903, 905,** and **907** may be moved together, or may be moved independently, depending on the arrangement of substrates employed. Microfluidic channel **901** contains a fluid. As shown in FIG. 9B, microfluidic channel **903** contains droplets **906** within an immiscible fluid.

As shown in FIG. 9B, microfluidic channel **913** contains droplets **906** and **904**. Droplet **904** contains the fluid from microfluidic channel **901,** and the fluid in microfluidic channel **901** is immiscible with the fluid in microfluidic channel **913**. Microfluidic channel **913** was aligned with microfluidic channel **901** for a span of time to only allow for a small volume of fluid to pass from microfluidic channel **901** into microfluidic channel **913**. This small volume of fluid formed into a droplet **904** within microfluidic channel **913,** which contains a fluid immiscible with the droplets in microfluidic channel **903** and the fluid in microfluidic channel **901**. Alignment may occur to allow for a small volume of fluid to pass between microfluidic channels to generate a droplet in the receiving microfluidic channel. As shown in FIG. 9B, microfluidic channel **913** is aligned with microfluidic channel **903** so that a portion of fluid spans an air gap **902** to thereby flow fluid and droplets **906** from microfluidic channel **903** into microfluidic channel **913**. Microfluidic channel **913** may slide or move to align with microfluidic channels **901, 903, 905,** and **907**. Microfluidic channel **913** can be slid to align with any of the microfluidic channels, or microfluidic channel **913** can be positioned so as not to align with any microfluidic channel, and therefore does not receive fluid or droplets. Microfluidic channel **913** may align with microfluidic channel **919**. Microfluidic channel **919** may be movable or may be stationary. Alignment between microfluidic channel **913** and microfluidic channel **919** allows for flow between the two channels.

As shown in FIG. 9B, microfluidic channel **933** is aligned with microfluidic channel **919** to allow fluid to flow between the two microfluidic channels. Microfluidic channel **933** can be slid to disengage from microfluidic channel **919**. A chamber **935** can be slid to engage with microfluidic channel **919** to thereby allow fluid to flow from microfluidic channel **919** into chamber **935**. Chamber **935** can be a waste chamber. As shown in FIG. 9B, microfluidic channel **933** can align with channels in channel plate **941** to deliver fluid from microfluidic channel **933** to the channels in the channel plate **941**. In some embodiments, channel plate 941 contains branched channels. Channel plate **941** can contain any number of channels. Additionally, channel plate **941** can align with channel plates **950** and **960.**

Droplet Formation by Separate Droplet Generation Module

In other embodiments, droplets are formed using a separate droplet generation module. The droplet generation module can be fluidically coupled to the system so that generated droplets can directly flow into different channels of the system. Alternatively, the droplet generation module can be a separate component in which droplets are generated and collected in a vessel and then separately loaded into systems of the invention. Droplet generation may be accomplished by numerous techniques. The droplets may be formed, for example, by dipping an open ended tube into a vessel. Exemplary sample acquisition devices are shown in McGuire et al. (U.S. patent application publication number 2010/0294048), the content of which is incorporated by reference herein in its entirety. Parameters such as channel diameter, dipping time, and system flow, may be adjusted so that wrapped droplets are formed of different volumes. In certain embodiments, a droplet contains no more than a single entitle, such as a single biological molecule or a single cell.

Methods of the invention involve forming sample droplets in which some droplets contain zero, one, or multiple entities, such as cells. In the preferred embodiment, the distribution of entities (e.g., cells) within droplets obeys the Poisson distribution. However, methods for non-Poisson loading of droplets are known to those familiar with the art, and include but are not limited to active sorting of droplets, such as by laser-induced fluorescence, or by passive one-to-one loading. The description that follows assumes Poisson loading of droplets, but such description is not intended to exclude non-Poisson loading.

In certain embodiments, the droplets are aqueous droplets that are surrounded by an immiscible carrier fluid. In other embodiments, the droplets are non-aqueous droplets surrounded by an immiscible fluid, such as oil droplets in a water continuous phase. Methods of forming such droplets are shown for example in Link et al. (U.S. patent application numbers 2008/0014589, 2008/0003142, and 2010/0137163), Stone et al. (U.S. patent number 7,708,949 and U.S. patent application number 2010/0172803), and Anderson et al. (U.S. patent number 7,041,481 and which reissued as RE41, 780), he content of each of which is incorporated by reference herein in its entirety.

In some embodiments, the sample fluid is aqueous, such as when employing a culture medium. The sample fluid is typically an aqueous buffer solution, such as ultrapure water (e.g., 18 mega-ohm resistivity, obtained, for example by column chromatography), 10 mM Tris HCl and 1 mM EDTA (TE) buffer, phosphate buffer saline (PBS) or acetate buffer. Any liquid or buffer that is physiologically compatible with the sample can be used. In the preferred embodiment, the sample fluid comprises cell medium, as disclosed above. The sample fluid comprises the necessary components to ensure cell health and growth. As discussed above, any laboratory produced or commercially available cell medium may be employed.

As discussed above, the carrier fluid is one that is immiscible with the sample fluid. The carrier fluid can be a non-polar solvent, decane (e g., tetradecane or hexadecane), fluorocarbon oil, silicone oil or another oil (for example, mineral oil). In a preferred embodiment of the invention, the carrier fluid has a high surface tension and therefore, is retained a microfluidic channel at an open end. In an aspect of the invention, the carrier fluid forms a meniscus at the open end of the microfluidic channel, caused by surface tension. The meniscus can be either convex or concave, depending on the carrier fluid and the surface of the microfluidic channel.

Key elements for using microfluidic channels to process droplets include producing droplet of the correct volume, producing droplets at the correct frequency and bringing together a first stream of sample droplets with a second stream of sample droplets in such a way that the frequency of the first stream of sample droplets matches the frequency of the second stream of sample droplets. In some embodiments of the present invention, gravitational forces control the flow rate within the device.

Methods for producing droplets of a uniform volume at a regular frequency are well known in the art. One method is to generate droplets using hydrodynamic focusing of a dispersed phase fluid and immiscible carrier fluid, such as disclosed in U.S. Publication No. US 2005/0172476 and International Publication No. WO 2004/002627. Feedback on the infusion rates of the carrier fluid and the dispersed fluid provides droplets that are uniform in size and generated at a fixed frequency over arbitrarily long periods of time.

Aspects of the invention may employ the use of a microfluidic droplet generator device. The droplet generator device is configured to be in fluid communication with the multichannel system of the invention. For example, see US Patent Application 20140017150, which is incorporated by reference. The generator device comprises a substrate; a microfluidic channel formed in the substrate; a fluid outlet in fluid communication with the microfluidic channel; and a mechanical element configured such that vibration of the mechanical element causes droplet dispensing from the fluid outlet. It should be appreciated by one of skill in the art that droplets can be generated by alternative methods and techniques.

Droplet Pick-up

Droplets of the invention may be picked-up or transferred from a vessel to a microfluidic channel. A vessel may include any structure that contains fluids or droplets, for example, wells within a well plate. Without being bound by theory, if a microfluidic channel is sufficiently narrow and the liquid adhesion to the microfluidic channel wall is sufficiently strong, surface tension can draw liquid up the microfluidic channel in a phenomenon known as capillary action. The height to which the column is lifted is given by:

$$h = \frac{2 \Upsilon_{la} \cos\Theta}{pgr}$$

where h is the height the liquid is lifted, $\Upsilon_{la}$ is the liquid-air surface tension, $p$ is the density of the liquid, r is the radius of the capillary, g is the acceleration due to gravity, $\Theta$ is the angle of contact described above. In an aspect of the invention, microfluidic channels can be positioned at or near the surface of a liquid and draw the liquid up into the microfluidic channel.

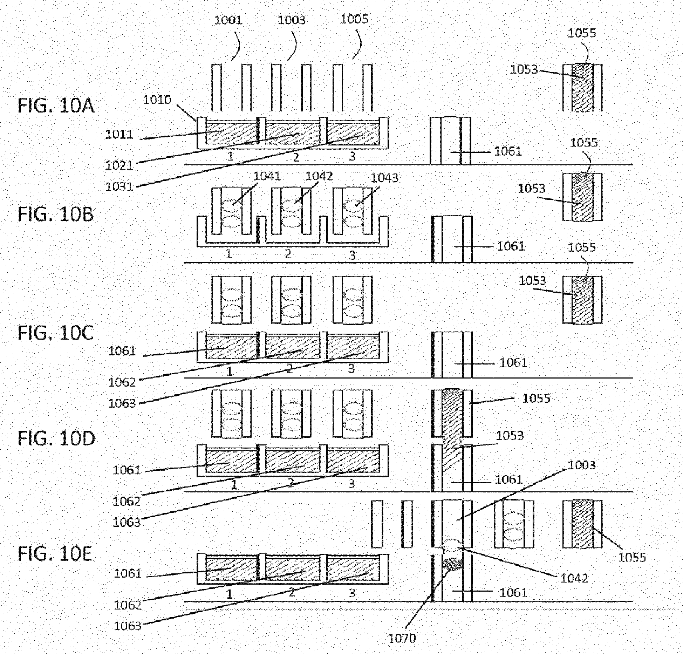

As shown in FIG. 10A, microfluidic channels **1001, 1003,** and **1005** are positioned proximate to substrate **1010.** Substrate **1010** contains open microfluidic channels **1, 2,** and **3.** Fluids **1011, 1021,** and **1031** are contained within open microfluidic channels **1, 2,** and **3** of substrate **1010.** Fluids **1011, 1021,** and **1031** may contain droplets. As shown in FIG. 10B, microfluidic channels **1001, 1003,** and **1005** are placed at or near the surface of fluids **1011, 1021,** and **1031** and draws up fluids **1011, 1021,** and **1031** into microfluidic channels **1001, 1003,** and **1005.** Open microfluidic channels **1, 2,** and 3 are emptied of fluid. Fluids **1011, 1021,** and **1031** may contain droplets **1041, 1042,** and **1043,** that are also drawn up into microfluidic channels **1001, 1003,** and **1005** or the up-down movement of channels **1001, 1003,** and **1005** in fluids **1011, 1021,** and **1031** may result in formation of droplets **1041, 1042,** and **1043** within channels **1001, 1003,** and **1005.**

In FIG. 10C, the microfluidic channels **1001, 1003,** and **1005** are positioned proximate to open microfluidic channels **1, 2,** and **3.** Open microfluidic channels **1, 2,** and **3** are replenished with fluids **1061, 1062,** and **1063.** Fluids **1061, 1062,** and **1063** may be the same as fluids **1011, 1021,** and **1031,** or fluids **1061, 1062,** and **1063** may be different. Similar to FIG. 10B, microfluidic channels **1001, 1003,** and **1005** are placed at or near the surface of fluids **1061, 1062,** and **1063** and draws up fluids **1061, 1062,** and **1063** into microfluidic channels **1001, 1003,** and **1005.** Open microfluidic channels **1, 2,** and **3** are emptied of fluid. Fluids **1061, 1062,** and **1063** may contain droplets that are also drawn up into microfluidic channels **1001, 1003,** and **1005** or the up-down movement of channels **1001, 1003,** and **1005** in fluids **1061, 1062,** and **1063** may result in formation of droplets within channels **1001, 1003,** and **1005.**

FIG. 10D shows the alignment of microfluidic channel **1061** with microfluidic channel **1055.** Microfluidic channel **1055** contains fluid **1053.** Aligning microfluidic channel **1061** and microfluidic channel **1055** causes fluid **1053** to flow from microfluidic channel **1055** into microfluidic channel **1061.** The volume of fluid **1053** can be of any size; whereas a small volume causes a droplet to form. As shown in FIG. 10E, after fluid **1053** has flowed into microfluidic channel **1061,** microfluidic channel **1003** aligns with microfluidic channel **1061** to allow for droplets **1042** to flow into microfluidic channel **1061.** The fluid **1055** may form a droplet and, using techniques disclosed herein, merges with droplet **1042** to form droplet **1070** (discussed below).

Droplets and the Microfluidic System

The microfluidic device of the invention may be utilized to flow droplets within microfluidic channels. As discussed in detail above, the microfluidic channels of the present invention can be aligned to allow the flow of fluids therebetween. In some embodiments of the present invention, the microfluidic system may be utilized to flow droplets within and between microfluidic channels. The droplets will typically be flowing in a carrier fluid, such as an oil.

In certain embodiments of the present invention, the fluid within the microfluidic channel contains droplets. As discussed above, droplets may be composed or various fluids and various components. As discussed previously, the microfluidic channels of the invention can be slid to align in order to select the path of the droplets within the microfluidic system. As the droplets flow through the channels, flow can be stopped within a microfluidic channel by misalignment with another microfluidic channel. Flow may resume once the microfluidic channel is aligned with another microfluidic channel such that flow occurs therebetween.

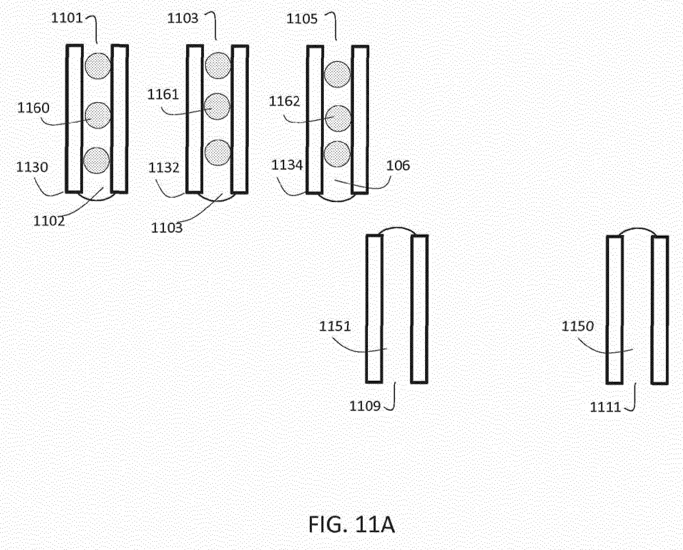

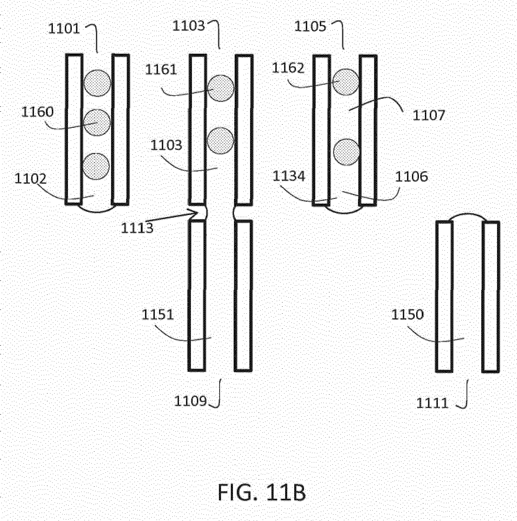

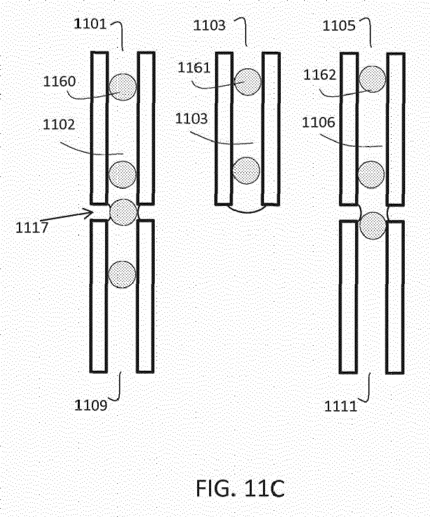

FIGS. 11A-11C depict a multi-channel system configured to allow microfluidic channels to slide or move relative to each other to alter alignment of the channels. FIG. 11A shows multiple microfluidic channels **1101, 1103,** and **1105** which are open at ends **1130, 1132,** and **1134.** Microfluidic channels **1101, 1103,** and **1105** each may contain a fluid; for example microfluidic channel **1101** contains fluid **1102.** Fluids **1102, 1103,** and **1106** maybe composed of the same components or may be composed of differing components. Each fluid **1102, 1103,** and **1106** is retained in the microfluidic channel by forces such as surface tension. As discussed above, an aspect of the invention is that fluid does not flow from the microfluidic channel unless aligned with another microfluidic channel. Also, each microfluidic channel **1101, 1103,** and **1105** contains droplets **1160, 1161,** and **1162.** As discussed above, droplets maybe composed of various fluids and components. Additionally, the droplets **1101, 1103,** and **1105** may comprise the same materials and components, or they droplets may comprise differing materials and components. Additionally, microfluidic channels **1101, 1103,** and **1105** may be slidable or moveable together or independent of one another. FIG. **11A** also depicts microfluidic channels **1109** and **1111** which are open ended at **1140** and **1141.** It should be appreciated that microfluidic channels **1109** and **1111** are shown in FIG. **11**A to contain fluids **1150** and **1151.** However, it should also be appreciated that microfluidic channels **1109** and **1111** may not contain fluids. Microfluidic channels **1109** and **1111** may be moved independent of one another or may be moved together. It is an aspect of the invention that microfluidic channels may be arranged in any configuration and manner. As shown in FIG. **11A,** microfluidic channels **1109** and **1111** are positioned to be disengaged from microfluidic channels **1101, 1103,** and **1105.** In this positioning of the microfluidic channels, microfluidic channels **1101, 1103,** and **1105** are prevented from flowing fluid due to the physical properties of the microfluidic channel and the immiscible fluid, e.g. surface tension, as discussed above.

Microfluidic channels **1109** and **1111** may be slid or moved to align with any of the microfluidic channels **1101, 1103,** or **1105.** FIG. 11B depicts microfluidic channels **1109** and **1111** that has been moved or slid relative to microfluidic channels **1101, 1103,** or **1105.** As shown in FIG. 11B, microfluidic channel **1109** has been slid to engage at least one of microfluidic channels **1101, 1103,** and **1105.** It should be appreciated that the moving or sliding of microfluidic channel **1109** or **1111** may involve movement in any plane or direction. In FIG. 11B, microfluidic channel **1103** is aligned with microfluidic channel **1109.** The alignment may cause an air gap **1113.** Also, as discussed above, it is not necessary for the microfluidic channels to be aligned so that the microfluidic channels are flush. Rather, an air gap **1113** may be present between the two microfluidic channels. The alignment of microfluidic channels **1103** and **1109** forms an air gap at **1113.** The formation of the air gap **1113** results in a portion of fluid spanning air gap **1113** and allows for fluid **1103** and droplets **1161** to flow from microfluidic channel **1103** into microfluidic channel **1109.** In this positioning, microfluidic channel **1109** receives fluid **1103** and droplets **1161** from microfluidic channel **1103,**

The microfluidic channels of the invention may be slid or move in several iterations. For example, as shown in FIG. 11C, microfluidic channel **1109** has been slid to align with microfluidic channel **1101.** As discussed previously, microfluidic channel **1109** was aligned with microfluidic channel **1103** and received fluid **1103** and droplets **1161.** Microfluidic channel **1109** now contains fluid **1103** and fluid **1102** and droplets **1161** and **1160.** In this embodiment, the components of the two different microfluidic channels are mixed. Additionally, as shown in FIG. 11C, microfluidic channel **1111** is aligned with microfluidic channel **1105.** Microfluidic channels **1109** and **1111** can be slid or moved at the same time, or independently of each other, depending on the configuration of microfluidic channels **1109** and **1111** and their respective substrates.

Multichannel systems of the invention may include numerous channels aligned in various planes of space. For example, FIGS. 11A - 11C serve to illustrate how two levels of microfluidic channels can align to direct the flow of droplets within a microfluidic system. It should be appreciated that different system architectures within the scope of the invention.

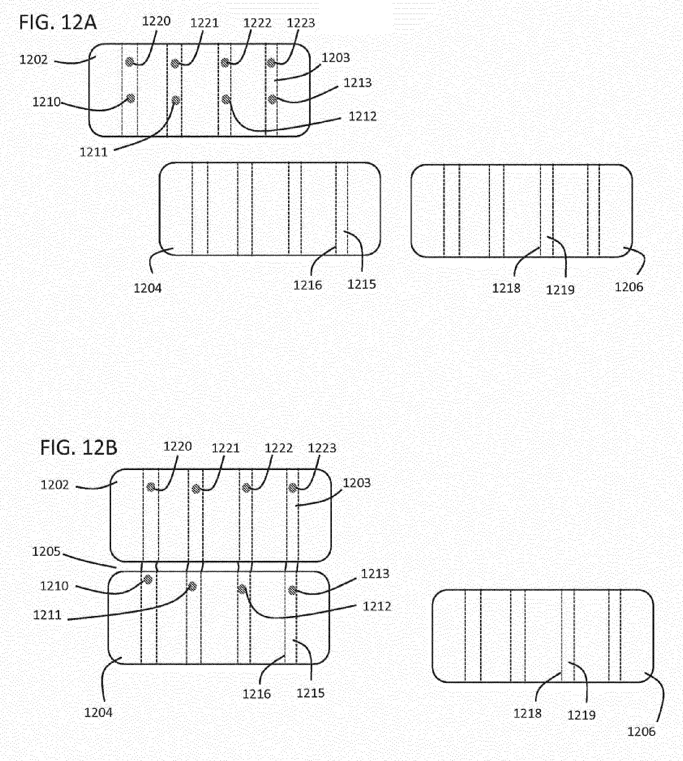

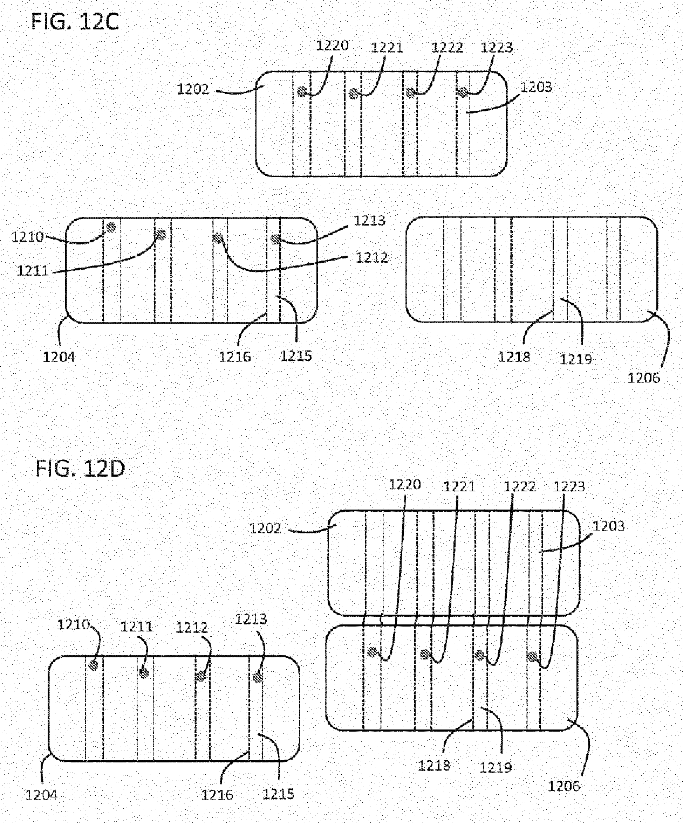

In preferred embodiments, as depicted in FIGS. 12A-12C, the channel plates of the invention transfer droplets and control fluids in a high through put workflow. As shown in FIG. 12A, a channel plate **1202** contains several channels **1203.** In the channels **1203** are droplets **1210, 1211, 1212,** and **1213.** Channels **1203** also contain droplets **1220, 1221, 1222,** and **1223.** Channel plates **1204** and **1206** are not aligned with channel plate **1202,** and therefore fluid does not flow between channel plates. As shown in FIG. 12B, channel plate **1202** and **1204** are aligned, so that the channels **1203** and channels **1215** align. When aligned, air gap **1205** is formed. The fluids in channels **1203** and channels **1215** bridge the air gap, allowing fluid to flow there between. Droplets **1210, 1211, 1212,** and **1213** flow into channels **1215.** As shown in FIG. 12C, channel plates **1202** and **1204** are misaligned, and droplets **1220, 1221, 1222,** and **1223** are retained in channel plate **1202.**

As shown in FIG. 12D channel plate **1202** aligns with channel plate **1206,** and the fluids in the channel plates bridge the air gap 1230 allowing fluid to flow there between. Droplets **1220, 1221, 1222,** and **1223** flow into channels **1218.** It should be appreciated that in the system depicted in FIGS. 12A-12D, a large number of droplets can be distributed across a plurality of channel plates in a high through put manner. Using the system depicted in FIGS. 12A-12D, a single droplet can be introduced into a channel, wherein the channel contains fluids for needed reactions in the droplet. Using this high throughput workflow, hundreds, thousands, and tens of thousands of assays can be completed quickly.

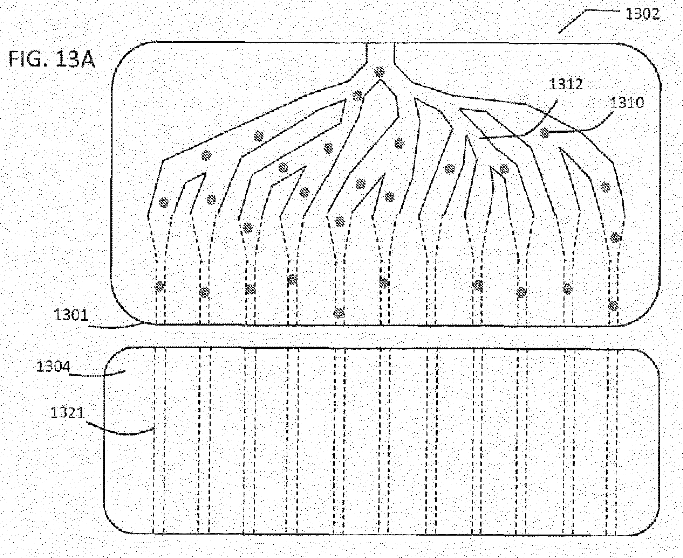

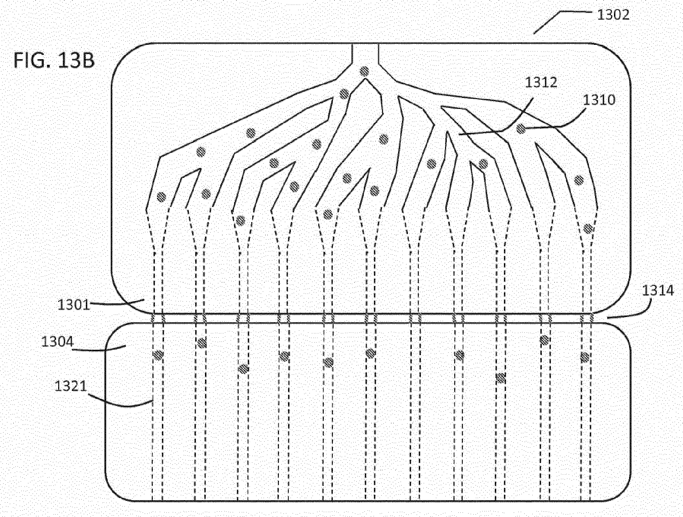

As shown in FIG. 13A, channel plate **1302** contains a plurality of droplets **1310** within channels **1312.** Channel plate **1302** contains a plurality of branched channels **1312** to allow for distribution of droplets within a channel plate system. It should be appreciated that any number of channels may be formed in a channel plate. Channel plate **1304** contains channels **1321.** When channel plate **1302** and channel plate **1304** are not aligned, fluid does not flow between the channels plates. As shown in FIG. 13B, channel plate **1302** and channel plate **1304** are aligned to create air gap **1314.** Fluid in channel plate **1302** bridges the air gap and a single droplet **1310** flow into each channel **1321** in channel plate **1304.** It should be appreciated that the time during alignment can be varied, thereby controlling the number of droplets that enter channels **1321.**

Droplet Merging

Systems of the invention can also be used for droplet merging or coalescing. The fluidic droplets may be of unequal size in certain cases. In certain cases, one or more series of droplets may each consist essentially of a substantially uniform number of entities of a species therein (i.e., molecules, cells, particles, etc.). The fluidic droplets may be coalesced to start a reaction, and/or to stop a reaction, in some cases. For instance, a reaction may be initiated when a species in a first droplet contacts a species in a second droplet after the droplets coalesce, or a first droplet may contain an ongoing reaction and a second droplet may contain a species that inhibits the reaction. Embodiments of the invention are directed to alignment of microfluidic channels to promote the coalescence of fluidic droplets.

In embodiments in which droplets do not contain cells, droplets may coalesce, for example, upon application of an electric field. An aspect of the invention incorporates the application of an electric field. The applied electric field may induce a charge, or at least a partial charge, on a fluidic droplet surrounded by an immiscible fluid. Upon the application of an electric field, for example by producing a voltage across electrodes using a voltage source, droplets are induced to assume opposite charges or electric dipoles on the surfaces closest to each other, causing the droplets to coalesce.

In another aspect of the invention, droplets may fuse by joining the fluid between two droplets, which may occur due to the charge-charge interactions between the two fluids. The creation of the bridge of fluid between the two droplets thus allows the two droplets to exchange material and/or coalesce into one droplet. Thus, in some embodiments, the invention provides for the coalescence of two separate droplets into one coalesced droplet in systems where such coalescence ordinarily is unable to occur, e.g., due to size and/or surface tension, etc. In some embodiments, one droplet is considerable smaller than another droplet. The smaller droplet merges or coalesces with the larger droplet to lower the internal pressure within the smaller droplet.

In droplets containing cells, use of electrodes or electric fields to merge droplets may need to be avoided. Coalescing of droplets without the application of an electric field is described by Xu et al., "Droplet Coalescence in Microfluidic Systems," Micro and Nanosystems, 2011, 3, 131-136, which is incorporated by reference. The method described by Xu et al. merges droplets by allowing two droplets to have close contact with each other, causing the liquids of the two droplets to form a thin bridge between the two droplets. The methods described by Xu et al. are passive, where an external energy source is not needed. Passive merging can also be accomplished by channel geometry. See for example Gu et al., Int J Mol Sci. 2011; 12(4): 2572-2597, published online Apr 15,2011. Doi 10.3390/ijms12042572, which is incorporated by reference. For example, droplet merging consists of a widening channel follow by a narrower channel. In this geometry the droplet velocity decreases in the widening channel, after which it increases again upon entry in the narrow channel. Droplets close in proximity merge.

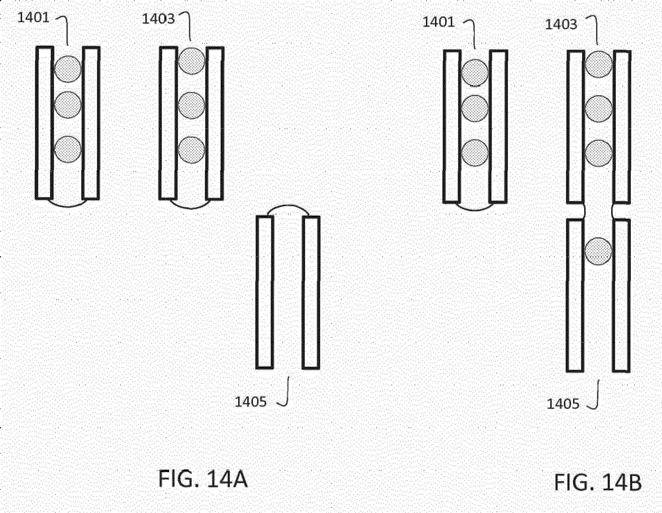

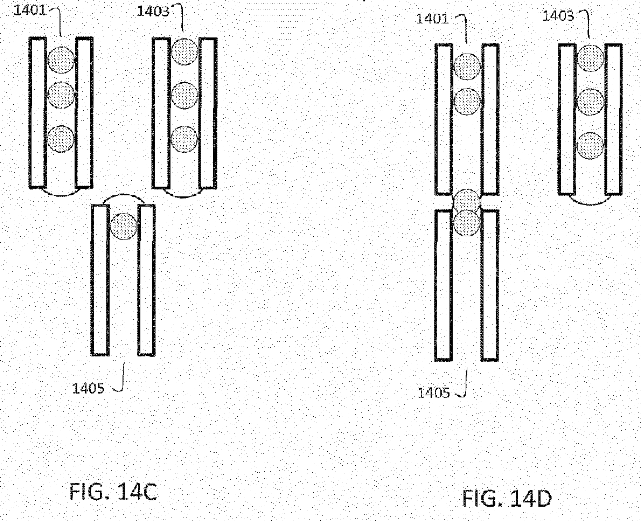

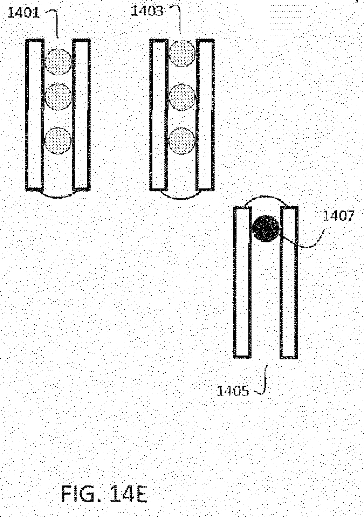

Another embodiment of the device is shown at FIG. 14A-14E. As shown in FIG. 14A, microfluidic channels **1401** and **1403** contain carrier fluid and at least one droplet. Microfluidic channel **1405** is not aligned with either microfluidic channel **1401** or **1403**. The carrier fluid and the droplets are not able to flow out of the open end of microfluidic channels **1401** or **1403**. FIG. 14B shows microfluidic channel **1403** aligned with microfluidic channel **1405**, thereby allowing flow of carrier fluid and droplets between microfluidic channel **1403** and microfluidic channel **1405**. FIG. 14C shows an embodiment in which microfluidic channel **1405** received a droplet when aligned to microfluidic channel **1403,** and the alignment is then disengaged. FIG. 14D shows microfluidic channel **1405** aligned with microfluidic channel **1401** to receive carrier fluid and a droplet from microfluidic channel **1401**. The droplets within microfluidic channel **1405** can placed close together, causing passive merging. Within microfluidic channel **1405**, the droplets are coalesced **1407**. As shown in FIG. 14E, droplet **1407** represents the coalesced droplet. As discussed above, coalesces may be accomplished by the application of an electric field. In droplets where cells are not present, electrodes may be located proximate to microfluidic channels to create an electric field to cause droplets to coalesce. In droplets in which cells are not present, passive merging can be accomplished by positioning droplets next to one another. In some embodiments in which droplets are present, aligning microfluidic channels to position droplets close together causes passive merging, as described above.

## Splitting of droplets

The microfluidic device of the present invention may be used to split droplets, by known methods. Droplet splitting has numerous applications and is particularly useful for culturing cells in droplets, which is discussed in more detail below. Examples include the splitting of droplets by directing the droplets towards an obstacle, such as is disclosed in U.S. patent application Ser. No. 11/024,228, filed Dec. 28, 2004, entitled "Method and Apparatus for Fluid Dispersion," by Stone, et al., now U.S. Pat. No. 7,708,949, issued May 4, 2010. Several methods for droplet splitting at a T-junction in a microfluidic system are known in the art. See for example, Link et al., (Phys. Rev. Lett. 2004, 92,4.) and Nie et al. (Anal. Chem. 2010, 82, 7852-7856). With an appropriate geometry it is possible to split a droplet in a ratio that is inversely proportional to flow resistances in each of the two branches of a T-junction. Other disclosed methods of droplet splitting are shown for example in International patent application publication number WO 2013/014215, which is herein incorporated by reference.

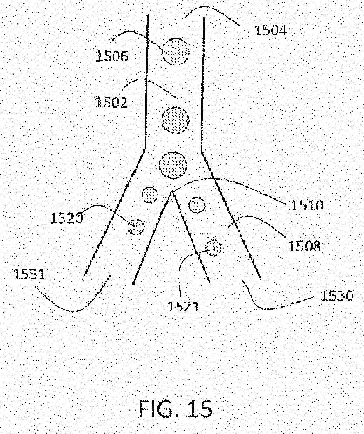

As shown in FIG. 15, a microfluidic channel **1504** can be configured to have a branched portion **1508.** An obstacle **1510** is positioned so droplets **1506** contact the obstacle and split into at least two different parts **1520** and **1521.** It should be appreciated that the open ends of the microfluidic channel **1531** and **1530** are able to align with other microfluidic channels or chambers (discussed below).

## Detectors

An aspect of the invention incorporates a detector within the devices and methods of the invention. The detection apparatuses can be optical or electrical detectors or combinations thereof. Examples of suitable detection apparatuses include optical waveguides, microscopes, diodes, light stimulating devices, (e.g., lasers), photo multiplier tubes, and processors (e.g., computers and software), and combinations thereof, which cooperate to detect a signal representative of a characteristic, marker, or reporter, and to determine and direct the measurement or the sorting action at a sorting module. Further description of detection modules and methods of detecting species in droplets are shown in Link et al. (U.S. patent application numbers 2008/0014589, 2008/0003142, and 2010/0137163).

## Droplet Collection

The multichannel system of the present invention is able to direct and manipulate the flow of fluids and droplets contained within the microfluidic system. An aspect of the invention involves the directing of droplets to microfluidic channels, waste chambers, or collection chambers. In a preferred embodiment of the invention, after processing within the microfluidic device, the droplets are directed to a collection chamber for removal, further processing, or detection. It should be appreciated that in conjunction with slidable microfluidic channels, slidable and moveable chambers can be incorporated into the devices and methods of the present invention.

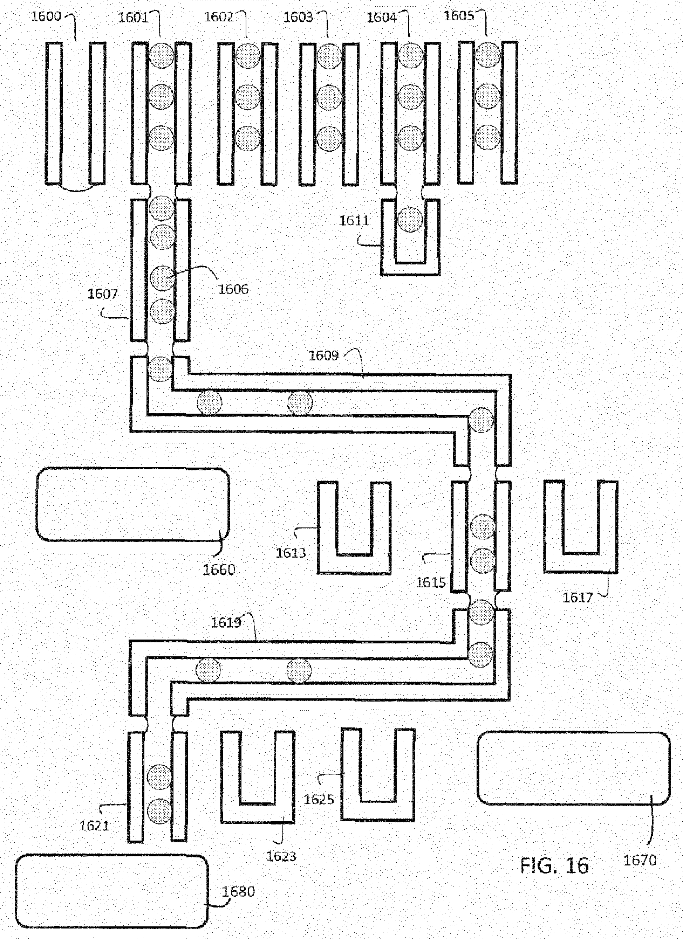

FIG. 16 depicts another embodiment of the invention. As shown in FIG. 16, a plurality of microfluidic channels, **1600, 1601, 1602, 1603, 1604,** and **1605** contain carrier fluid and droplets. Microfluidic channel **1601** is aligned with microfluidic channels **1607** to allow fluid and droplets to flow from microfluidic channels **1601** to microfluidic channels **1607** and then to microfluidic channels **1609**. Microfluidic channel **1603** is aligned with microfluidic channels **1611,** which can serve as a microfluidic channels and as a chamber. The chamber can be a waste chamber, a collection chamber, or other purpose chamber employed with the device and methods of the invention. As shown in FIG. **16,** microfluidic channels **1611** accepts fluid from microfluidic channels **1603,** and microfluidic channels **1611** serves as a waste chamber. Microfluidic channels **1600, 1601, 1602, 1603, 1604,** and **1605** may be attached to or positioned within a surface, wherein the surface is movable. It should be appreciated that microfluidic channels **1600, 1601, 1602, 1603, 1604,** and **1605** may be moved together or independently of one another. Microfluidic channel **1607** also may be slid or remain in a stationary position. Microfluidic channel **1611** may be slid or remain in a stationary position. Fluid and droplets flow into microfluidic channel **1609** when a microfluidic channel aligns with microfluidic channel **1609**. Microfluidic channel **1609** may be slidable or stationary. Fluid and droplets can flow from microfluidic channel **1609** to microfluidic channel **1619** when microfluidic channel **1615** is aligned there between. Microfluidic channel **1613** can align with microfluidic channel **1609** to divert the fluid and/or droplets to a waste chamber. Microfluidic channel **1617** can align with microfluidic channel **1617** to diver the fluid and/or droplets to a collection chamber. Channel plate **1660** can align with microfluidic channel **1609** to flow fluid and/or droplets into channels in channel plate **1660**. Channel plate **1660** can contain any number of channels, which can be branched, linear, nonlinear, etc. Channel plate **1670** can also align with microfluidic channel **1619**. Microfluidic channels **1621, 1623,** and **1625** can align with microfluidic channel **1619** to divert or direct the flow of droplets into another microfluidic channel, a waste chamber or a collection chamber. Alternatively, droplets can be diverted into an incubation chamber for further processing. Microfluidic channel **1621** can align and deliver fluid and/or droplets in channel plate **1680.**

Circulating Channel

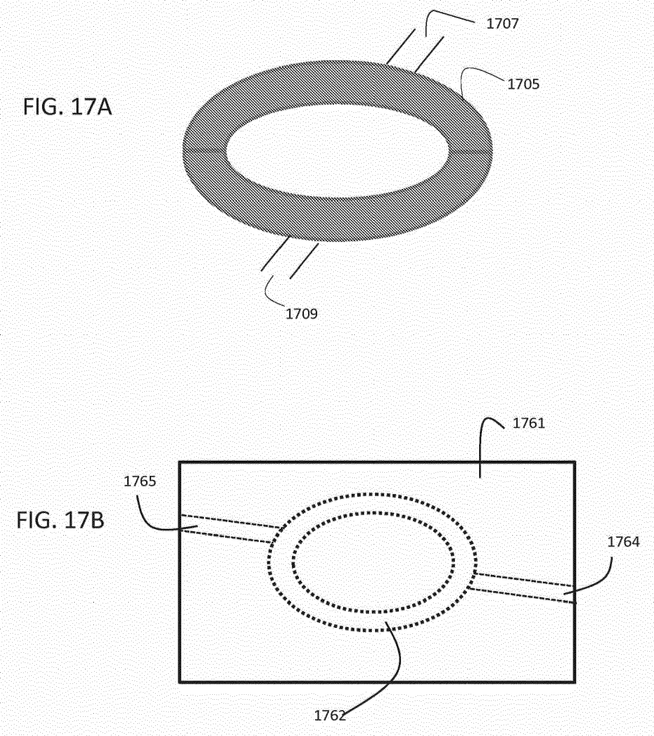

As discussed above, microfluidic channels may be nonlinear, or even contain branches. In some embodiments, microfluidic channels of the present invention may be substantially circular. See FIGS. 17A and 17B. As shown in FIG. 17A, fluid enters the circular portion **1705** through microfluidic channel **1707**. Fluid may exit at microfluidic channel **1709**. FIG. 17B depicts a microfluidic channel in substrate **1761** that has a circular portion **1762**. Fluids may enter the circular portion through microfluidic channel **1765** and may exit through microfluidic channel **1764**. In some embodiment of the invention, the circulating channel is used so that cell culturing can be performed in systems of the invention. Cells are held in microfluidic aqueous droplets that are separated from one another by silicone oil, or any immiscible fluid. These droplets are then introduced into the circular microfluidic channel. The droplets are held in a circular cross-section channel which has a rotating inner wall and a stationary outer wall. Rotation is achieved by a rotor or similar device. In that manner, the droplets can circulate for as long as is required, for example for culturing of a cell in the droplet. Waste material from the cells diffuses to the lower portion of the circular path. An outlet channel configured at the lower portion of the circular path removes the waste material. An inlet channel configured to deliver fluids into the circular path allows for the introduction of additional fluids, such as cell medium.

In a preferred embodiment of the invention, cells are introduced into a circular channel and flowed in the circular path. Waste from the cells diffuses out of the droplets into the immiscible fluid. The waste from the cells is removed and fluids are replenished to approximately maintain the volume in the circular path. For example, if the droplets contain cells and cell medium, the cell medium may be replenished by coalescing with a droplet containing cell medium.

Thermal Regulator

In another aspect of the invention, a portion of the multichannel system comprising one or more microfluidic channels is in thermal contact with a thermal regulator. The thermal regulator can be any device that regulates temperature. This includes, for example, resistive wires that heat up when a voltage is applied (such as those used in toasters), resistive heaters, fans for sending hot or cold air toward the isolated portion, Peltier devices, IR heat sources such as projection bulbs, circulating liquids or gases in a contained device, and microwave heating.

Thermal contact between a portion of the multichannel system and the thermal regulator provides thermal regulation of fluidic samples or droplets contained therein for incubation and/or regulation of biochemical or chemical reactions. The ability of the thermal regulator to be programmed for different temperatures and incubation times, together with other aspects of the invention to control the introduction of samples, reactants and other reagents into the microfluidic channels provides the ability to control reaction times, temperatures, and reaction conditions within the microfluidic channels. For example, a portion of the multichannel system is in contact with a heat spreader of the thermal regulator. There may be an air gap between the portion of the microfluidic device and the heating element of the thermal regulator. The microfluidic device can be secured to the thermal regulator by one or more bolts, screws, pins, clips, brackets, or other such securing devices.

The thermal regulator can be an electrical apparatus comprising one or more temperature sensors, e.g., thermocouples, thermistors, RTDs, and one or more regulators configured for temperature regulation, incubating, or thermal cycling a portion of an attached microfluidic device. In turn, the fluidic biological or chemical samples introduced into the portion of the microfluidic device are heated, incubated, cooled, or thermal cycled in repetitive fashion in order to carry out one or more of a number of biochemical or chemical procedures or processes.

In another embodiment, the thermal regulator is configured into separate thermal zones, or stations, and each zone comprising a separate thermal regulator, one or more temperature sensors, and a regulator dedicated to each zone for the separate thermal control of that zone. For example, an embodiment of the invention may include four individually controlled thermal zones each configured to a portion of a microfluidic device.

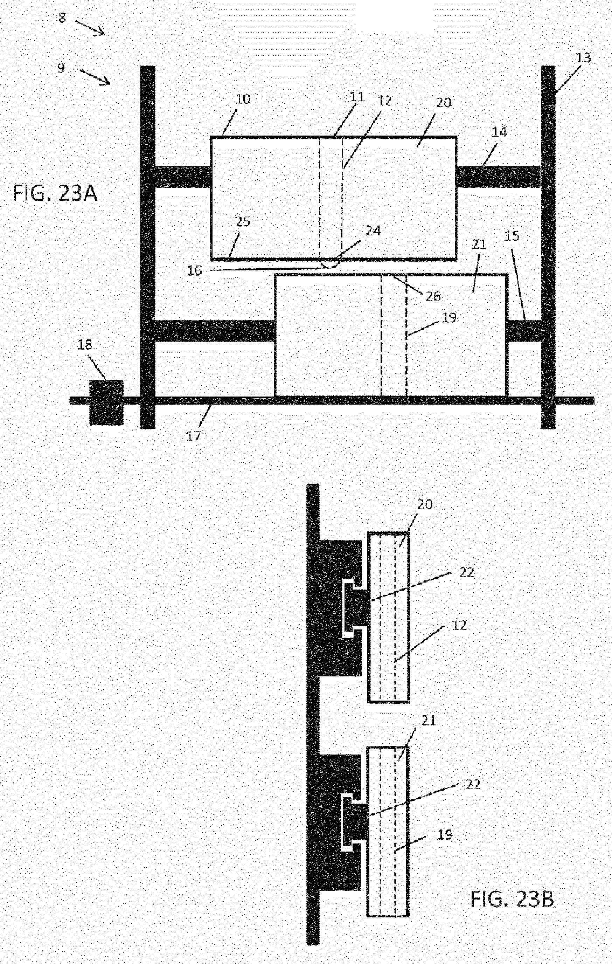

In some embodiments of the invention, as shown in FIGS. 23A and 23B, a microfluidic system 8 comprises a first substrate 20 with at least one reservoir 12 in fluid communication with an upper opening 11 at an upper surface 10 of the first substrate 20 and a lower opening 24 at a lower surface 25 of the first substrate 20, wherein the lower opening 24 is dimensioned such that a liquid 16 held in the reservoir 12 is prevented from flowing through the lower opening 24 and into ambient atmosphere by surface tension. The microfluidic system 8 also comprises a mechanical subsystem 9 supporting the first substrate 20 in an orientation such that liquid dispensed to the upper opening 11 flows into the reservoir 12. The mechanical subsystem 9 comprises an upper rail 14 and a lower rail 15. The microfluidic system 8 also comprises a second substrate 21 coupled to 22 and controlled by the mechanical subsystem 9, the second substrate 21 comprising a receptacle 19 open to a surface 26 of the second substrate 21, wherein operation of the mechanical subsystem 9 while a liquid 16 is held within the reservoir 12 brings the second substrate 21 into contact with a surface of the liquid 16, causing the liquid 16 to flow into the receptacle 19.

In some embodiments the systems are coupled or operably linked to a mechanical subsystem 9 comprising a chassis 13. A substrate of the invention may be operably coupled to a drive rail 17 which is operably connected to a motor 18, such that operation of the motor causes movement of the drive rail 17 which slides a substrate 21 which is coupled to a rail 15 by an attachment configuration 22.

Robotic Stages

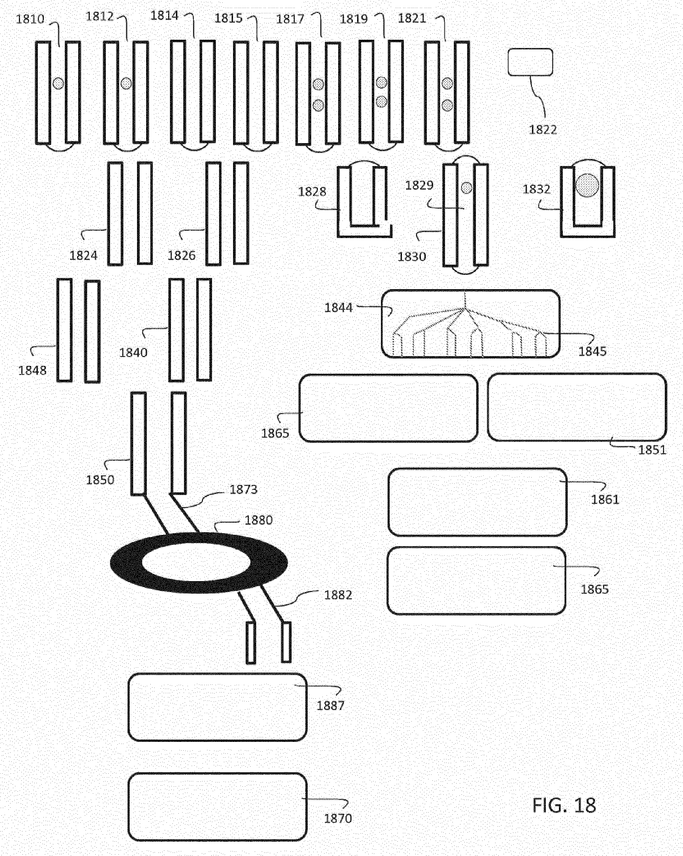

The systems of the invention can be controlled by robotic stages. As seen in FIGS. 16 and 18, various components comprise the microfluidic devices of the invention. It should be appreciated that the various components can be controlled by robotics. In an exemplary system of the invention, the microfluidic components are controlled by robotics, thereby allowing for assays to be further controlled through alignment and misalignment of the components. A system may include a data processor, a motion controller, a robotic arm assembly, a monitor element, a central processing unit, a microliter plate of source material, a stage housing, a robotic arm, a stage, a pressure controller, a conduit, a mounting assembly, a pin assembly, and microfluidic component elements. Stages and arms of the robotic assembly are moveable in the x-y-z planes.

The data processor can be a conventional digital data processing system such as an IBM PC compatible computer system that is suitable for processing data and for executing program instructions that will provide information for controlling the movement and operation of the robotic assembly. It will be apparent to one skilled in the art that the data processor unit can be any type of system suitable for processing a program of instruction signals that will operate the robotic assembly that is integrated into the robotic housing. Optionally the data processor can be a micro-controlled assembly that is integrated into the robotic housing. In further alternative embodiments, the system need not be programmable and can be a singleboard computer having a firmware memory for storing instructions for operating the robotic assembly.

The controller can be electronically coupled between the data processor and the robotic assembly. The controller is a motion controller that drives the motor elements of the robotic assembly for positioning the robotic arm at a selected location. Additionally, the controller can provide instructions to the robotic assembly to direct the pressure controller to control the volume of fluid or gas injected into the system. It should be appreciated that any design of a robotic assembly could be used in the present invention. The design and construction of robotic assemblies are well known in the art of electrical engineering, and any controller element suitable for driving the robotic assembly can be used. Accordingly, it will be apparent to one of skill in the art that alternative robotic systems can be used.

Assays

The systems of the invention can be used for any process that involves manipulation and movement of small volumes of fluid (e.g., micro scale, nano scale, etc.). In certain embodiments, systems of the invention are used for chemical synthesis reactions or biological or chemical assays, such as sample preparation and analysis in a variety of fields in the art, including without limitation for many fields such as DNA sequencing, microarray sample preparation, genotyping, gene expression, biodefense, food monitoring, forensics, proteomics and cell biology. Assays involving the amplification of nucleic acids may be performed in the device of the present invention. Thus, assays applying to digital amplification techniques and multiplex PCR in droplets can be achieved using the device of the present invention. See for example, United States Patent Application 20110244455, herein incorporated by reference.

Numerous processes may be completed within the device of the present invention. Nucleic acids, proteins, lipids, etc. may be processed and analyzed with the present invention and methods. However, it should be appreciated that any material or species may be enveloped in a droplet and processed in the device of the invention. For example, samples may be obtained from animals (including humans) and encompass fluids, solids, tissues, and gases. Biological samples include, without limitation, cells and any components thereof, blood products, such as plasma, serum and the like, proteins, peptides, amino acids, polynucleotide, lipids, carbohydrates, and any combinations thereof. The sample may include chemicals, organic or inorganic, used to interact with the interactive material. Environmental samples include environmental material such as surface matter, soil, water, crystals and industrial samples.

Generally, nucleic acid can be extracted from a biological sample by a variety of techniques such as those described by Maniatis, et al., Molecular Cloning: A Laboratory Manual, Cold Spring Harbor, N.Y., pp. 280-281 (1982). Nucleic acid molecules may be single-stranded, double-stranded, or double-stranded with single-stranded regions (for example, stem- and loop-structures). Nucleic acid molecules can be synthetic or derived from naturally occurring sources. As known in the art, nucleic acid molecules are isolated from a biological sample containing a variety of other components, such as proteins, lipids and non-template nucleic acids. Nucleic acid template molecules can be obtained from any cellular material, obtained from an animal, plant, bacterium, fungus, or any other cellular organism. Nucleic acid molecules may be obtained from a single cell. Biological samples for use in the present invention include viral particles or preparations. Nucleic acid molecules can be obtained directly from an organism or from a biological sample obtained from an organism, e.g., from blood, urine, cerebrospinal fluid, seminal fluid, saliva, sputum, stool and tissue. Any tissue or body fluid specimen may be used as a source for nucleic acid for use in the invention.

Nucleic acid molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA. In certain embodiments, the nucleic acid molecules are bound as to other target molecules such as proteins, enzymes, substrates, antibodies, binding agents, beads, small molecules, peptides, or any other molecule and serve as a surrogate for quantifying and/or detecting the target molecule.

Any suitable PCR methodology or combination of methodologies may be utilized in the droplet-based assays disclosed herein, such as aliele-specific PCR, assembly PCR, asymmetric PCR, digital PCR, endpoint PCR, hot-start PCR, in situ PCR, intersequence-specific PCR, inverse PCR, linear after exponential PCR, ligation-mediated PCR, methylation-specific PCR, miniprimer PCR, multiplex ligation-dependent probe amplification, multiplex PCR, nested PCR, overlap-extension PCR, polymerase cycling assembly, qualitative PCR, quantitative PCR, real-time PCR, RT-PCR, single-cell PCR, solid-phase PCR, thermal asymmetric interlaced PCR, touchdown PCR, or universal fast walking PCR, among others.

Methods for performing polymerase chain reaction (PCR) in droplets are shown for example in Link et al. (U.S. patent application numbers 2008/0014589, 2008/0003142, and 2010/0137163), Anderson et al. (U.S. Pat. No. 7,041,481 and which reissued as RE41,780) and European publication number EP2047910 to Raindance Technologies Inc. The content of each of which is incorporated by reference herein in its entirety. See also Brown et al. (U.S. Pat. Nos. 6,143,496 and 6,391,559) and Vogelstein et al. (U.S. Pat. Nos. 6,440,706, 6,753,147, and 7,824,889), the content of each of which is incorporated by reference herein in its entirety, relating to digital PCR (dPCR) as an alternative quantitation method in which dilute samples are divided into many separate reactions. The nucleic acids undergo the same thermal cycling and chemical reaction as the droplets passes through each thermal cycle as they flow through the channel. The total number of cycles in the device is easily altered by an extension of thermal zones or by the creation of a continuous loop structure. The sample undergoes the same thermal cycling and chemical reaction as it passes through N amplification cycles of the complete thermal device. The device and methods of the present invention have utility in droplet based digital PCR technology, as described in Link et al. (U.S. patent application numbers 2008/0014589, 2008/0003142, and 2010/0137163), Anderson et al. (U.S. Pat. No. 7,041,481 and which reissued as RE41,780) and European publication number EP2047910 to Raindance Technologies Inc, (the contents of each of which are incorporated by reference herein in their entireties). In this assay, a library droplet is merged with a template droplet which contains all the PCR reagents including genomic DNA except for the primers. After merging of the template and the primer library droplets the new droplet contains all the reagents necessary to perform PCR. The droplet is then thermal cycled to produce amplicons.

In some embodiments of the invention, the device and methods are instrumental in a polymerase chain reaction, or other methods of analyzing nucleic acids. In this embodiment, after amplification of nucleic acids, droplets are flowed to a detection module for detection of amplification products. The droplets may be individually analyzed and detected using any methods known in the art, such as detecting the presence or amount of a reporter. Generally, the detection module is in communication with one or more detection apparatuses.

In certain embodiments, amplified target are detected using detectably labeled probes. In particular embodiments, the detectably labeled probes are optically labeled probes, such as fluorescently labeled probes. Examples of fluorescent labels include, but are not limited to, Atto dyes, 4-acetamido-4'-isothiocyanatostilbene-2,2'disulfonic acid; acridine and derivatives: acridine, acridine isothiocyanate; 5-(2-aminoethyl)aminonaphthalene-1-sulfonic acid (EDANS); 4-amino-N-[3-vinylsulfonyl)phenyl]naphthatimide-3,5 disulfonate; N-(4-anilino-1-naphthyl)maleimide; anthranilamide; BODIPY; Brilliant Yellow; coumarin and derivatives; coumarin, 7-amino-4-methyleoumaiin (AMC, Coumarin 120), 7-amino-4-trifluoromethylcouluarin (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethytamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatosfilbene-2,2'-disulfonic acid; 5-[dimethylamino]naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron™ Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N,N'tetramethyl-6-carboxyrliodamine (TAMRA); tetramethyl rhodamine; tetramethyl rhodamine isothiocyanate (TRITC); riboflavin; rosolic acid; terbium chelate derivatives; Cy3; Cy5; Cy5.5; Cy7; IRD 700; IRD 800; La Jolta Blue; phthalo cyanine; and naphthalo cyanine. Preferred fluorescent labels are FAM and VIC (fluorescent label, commercially available from Applied Biosystems, Inc.). Labels other than fluorescent labels are contemplated by the invention, including other optically-detectable labels. See for example, United States Patent Application 20110244455.

Cell Culture Assays using Systems of the Invention

Devices and methods of the present invention have utility in the field of cell culturing. Cell culturing devices are commercially available, however, each of the currently available systems have at least, the following limitations: large size; high cost (particularly robotics); possible contamination, use of large scale cultures; treating cells with an enzyme; requiring complex software and using a large cell volume. Commercially available cell automation systems are developed by combining robotic stages, however, these systems are expensive given the cost of the robotic arms and the software used to drive them. There are a number of other automated cell systems commercially available, see for example the PANsys3000 system, which is a highly automated cell-culture system manufactured by Pan-SysTech; the CompacT SelecT system, which is an automated cell culture and assay- ready plating system manufactured by the Automation Partnership; Tecan, Inc. manufacturers several automated devices for cell culturing; Hamilton Robotics offers an extensive range of robotic systems for incorporation into cell culturing systems; and an automated cell culture system manufactured by MatriCal Bioscience.

Any type or kind of cell may be used in conjunction with the current invention. For example, induced pluripotent stem cells or iPS cells or iPSs, may be used in conjunction with the present invention. An iPS cell refers to a cell that has been reprogrammed from a somatic cell to a more pluripotent phenotype by any means of reprogramming. The culture environment within the device of the invention must be controlled and monitored to ensure the health of the cells. The specific culture conditions may vary depending on the cell type. However, most culture conditions consist of a suitable vessel containing a substrate or medium that supplies the essential nutrients (amino acids, carbohydrates, vitamins, and minerals), growth factors, hormones and gases ($O_2$, $CO_2$). Furthermore, the physicochemical environment (pH, osmotic pressure, temperature) must be regulated. Cells may be grown floating in the culture medium (suspension culture) or grown while attached to a solid or semi-solid substrate (adherent or monolayer culture).

Harvesting of cells can be accomplished by known methods in the art. Cells can be isolated from tissues for ex vivo culture in a variety of ways. For example, cells can be purified from blood; however, only the white cells are capable of growth in culture. Mononuclear cells can be released from soft tissues by enzymatic digestion with enzymes such as collagenase, trypsin, or pronase. Primary cells, cells cultured directly from a subject, have limited lifespan. After a certain number of population doublings, cells stop dividing but retain viability. There is the case of established or immortalized cell lines that have acquired the ability to proliferate indefinitely either through random mutation or deliberate modification.

Cells are grown and maintained at an appropriate temperature and gas mixture (typically, 37 °C, 5% $CO_2$ for mammalian cells), usually in a cell incubator. Culture conditions vary widely for each cell type, and variation of conditions for a particular cell type can result in different phenotypes. See for example, Tsai et al., "Isolation of human multipotent mesenchymal stem cells from second-trimester amniotic fluid using a novel two-stage culture protocol," Hum. Reprod.

(2004) 19 (6): 1450-1456. doi: 10.1093/humrep/deh279, where amniotic fluid-derived mesenchymal stem cells (AFMSCs) were cultured to confluence and shifted to osteogenic medium ($\alpha$-MEM supplemented with 10% FBS, 0.1 $\mu$mol/l dexamethason, 10 mmol/l $\beta$-glycerol phosphate, 50 $\mu$mol/l ascorbate) and adipogenic medium (a-MEM supplemented with 10% FBS, 1 $\mu$mol/l dexamethasone, 5 $\mu$g/ml insulin, 0.5 mmol/l isobutylmethylxanthine and 60 $\mu$mol/l indomethacin) for 3 weeks. For differentiation of neural cells, AFMSCs were incubated with $\alpha$-MEM supplemented with 20% FBS, 1 mmol/l $\beta$-mercaptoethanol, 5 ng/ml bFGF (Sigma, St Louis) for 24 h, and then treated with serum depletion for 5 h.

The cell medium provides the necessary nutrients, growth factors, and hormones for cell growths, as well as regulating the pH and the osmotic pressure of the culture. The cell culture medium according to the present invention is a medium allowing for and supporting growth of the animal cells thus cultured. Growth is understood as an increase in viable cell density during at least a certain period of the cell culture. According to the present invention, such definition of 'growth medium' is to be understood as being opposed to the term 'maintenance medium' in its usual meaning in the art. A maintenance medium is a cell culture medium which supports cell viability but which does not encourage cell growth. Often, such maintenance media do not contain essential growth factors such as transferrin, insulin, albumin and the like. See for example, cell culture medium related patents: US Patent 4,038,139, 1977; US Patent 7,258,998, 2007; US Patent App. 13/497,707, 2010; US Patent 8,338,177, 2012; and US Patent App. 13/695,002, 2011.

The growth medium controls the pH of the culture and buffers the cells in culture against fluctuations in the pH. This buffering may be achieved by including an organic (e.g., HEPES) or $CO_2$ bicarbonate based buffer. Control of pH is needed to ensure the growth and health of cells in culture. Most normal mammalian cell lines grow well at pH 7.4, and there is very little variability among different cell strains. However, some transformed cell lines have been shown to grow better at slightly more acidic environments (pH 7.0-7.4), and some normal fibroblast cell lines prefer slightly more basic environments (pH 7.4-7.7). Because the pH of the medium is dependent on the delicate balance of dissolved carbon dioxide ($CO_2$) and bicarbonate ($HCO_3$), changes in the atmospheric $CO_2$ can alter the pH of the medium. Therefore, it is necessary to use exogenous $CO_2$ when using media buffered with a $CO_2$ bicarbonate based buffer, especially if the cells are cultured in open dishes or transformed cell lines are cultured at high concentrations. While most researchers usually use 5-7% $CO_2$ in air, 4-10% $CO_2$ is common for most cell culture experiments. However, each medium has a recommended $CO_2$ tension and bicarbonate concentration to achieve the correct pH and osmolality.

The optimal temperature for cell culture largely depends on the body temperature of the host from which the cells were isolated, and to a lesser degree on the anatomical variation in temperature (e.g., temperature of the skin may be lower than the temperature of skeletal muscle). Overheating is a more serious problem than under heating for cell cultures; therefore, often the temperature in the incubator is set slightly lower than the optimal temperature. Most human and mammalian cell lines are maintained at 36°C to 37°C for optimal growth. Insect cells are cultured at 27°C for optimal growth; they grow more slowly at lower temperatures and at temperatures between 27°C and 30°C. Above 30°C, the viability of insect cells decreases, and the cells do not recover even after they are returned to 27°C. Avian cell lines require 38.5°C for maximum growth. Although these cells can also be maintained at 37°C, they will grow more slowly. Cell lines derived from cold-blooded animals (e.g., amphibians, cold-water fish) tolerate a wide temperature range between 15°C and 26°C. The consequences of deviating from the culture conditions required for a particular cell type can range from the expression of aberrant phenotypes to a complete failure of the cell culture.

Subculturing, or passaging, is the removal of the medium and transfer of cells from a previous culture into fresh growth medium, a procedure that enables the further propagation of the cell line or cell strain. Traditionally, to keep the cells at an optimal density for continued growth and to stimulate further proliferation, the culture has to divided and fresh medium supplied. For example, subculturing could be needed if a drop in pH is observed. A drop in the pH of the growth medium usually indicates a buildup of lactic acid, which is a by-product of cellular metabolism. Lactic acid can be toxic to the cells, and the decreased pH can be sub-optimal for cell growth.

As cells generally continue to divide in culture, they generally grow to fill the available area or volume. This can generate several issues: nutrient depletion in the growth media; changes in pH of the growth media; and accumulation of apoptotic/necrotic (dead) cells. Cell-to-cell contact can stimulate cell cycle arrest, causing cells to stop dividing, known as contact inhibition. Cell-to-cell contact can also stimulate cellular differentiation. Genetic and epigenetic alterations, with a natural selection of the altered cells potentially leading to overgrowth of abnormal, culture-adapted cells with decreased differentiation and increased proliferative capacity. Therefore, processing of the cells to ensure removal of harmful species and replenishment of cell medium is needed every 1-3 days, depending on the particular protocol.

Traditionally, cell viability is determined by staining the cells with trypan blue. As trypan blue dye is permeable to non-viable cells or death cells whereas it is impermeable to this dye. Stain the cells with trypan dye and load to haemocytometer and calculate % of viable cells. Cell viability is calculated as the number of viable cells divided by the total number of cells within the grids on the hemacytometer. If cells take up trypan blue, they are considered non-viable. It would be appreciated by one skilled in the art for the optical detection of cells containing trypan blue.

Cell culture entails growing cells in a growth medium under controlled temperature and atmosphere conditions. In the present invention, cells are encapsulated in droplets containing cell medium. The cells can be flowed from various chambers via the multichannel system of the invention. Microfluidic channels can be aligned to cause flow of the droplets into a humidifier chamber. For example, mammalian cells are grown in humidified atmosphere at 37° C and 5% $CO_2$, in cell culture incubators. Microfluidic channels can be aligned to deliver $CO_2$ to the humidifier chamber within the multichannel system by aligning microfluidic channels that seal, or by an inlet microfluidic channel configured to deliver liquids or gases to the chamber. It should be appreciated that alignment of microfluidic channels of the invention can be sealed to allow for the transport of gases. To replenish or re-suspend the cells in fresh growth medium, which could be required every 2-3 days, the droplets are flowed from a chamber into the microfluidic channels to be merged or coalesced with fresh growth medium. It should be appreciated that droplets containing cells during culturing may be retained within microfluidic channels or chambers of the present invention. After merging or coalesced with fresh growth medium, the droplets may be retained within microfluidic channels, or the cells may be diverted to a chamber.

In an embodiment of the invention, cells are grown in nanoliter-microliter droplets in cell medium that is replenished every 2-3 days. In some assays, cells may require splitting every 2-3 days. Media change involves adding one or more droplets of fresh media to a droplet of incubated cells and thereby partially replenishing growth media. Merging of droplets is discussed above. Cells are further incubated in the combined droplet or in smaller droplets generated by splitting the combined droplet. Cell subculture or splitting is achieved similarly to media change by combining (merging and mixing) a droplet of incubated cells and a droplet of fresh media, splitting the combined droplet, and repeating this procedure using the split droplet(s) until a desired cell concentration is reached. Final droplets are then incubated, while other droplets of suspended cells generated in the subculturing process are discarded. Incubation can be accomplished within the microfluidic channels of the device, or in chambers of the device. One or more thermal regulators can be employed to ensure proper temperature.

In a multiplexed assay, multiple droplets containing one kind or multiple kinds of cells are exposed to droplets containing one or multiple reagents and are assayed similarly to the assays described above. A multiplex device can also be used for multiplex cell culture, where cells can be grown and maintained in multiple droplets.

There are several ways of configuring the chambers for housing the droplets. In one configuration the chambers are external to the microfluidic device. Alternatively, the chambers could be integrated into the microfluidic device, and are in flow communication with the microfluidic channels of the microfluidic device. Signals from secondary droplets are detected using multiplexed detection instruments such as optical sensors, optical detectors comprising a light source and a photodetector, optical detectors that measure absorbance, fluorescence, epifluorescence, chemiluminescence, UV light detector, radiometric detector, scanning, imaging, and confocal microscopy detectors, CCD cameras, and microplate readers. The detection step is to detect or identify any reaction products formed by the cell assay, or to identify, monitor and count the cells if a cell culture is being performed to mention just a few. All waste liquid droplets generated during the assay are directed to the waste chamber. Chambers may contain wash solutions for flushing the microfluidic channels of the device between assays.

The devices and methods of the present invention have utility in the area of cell culturing. A multichannel device of the invention can be employed to culture cells in a controlled and stable environment. For example, FIG. 16 depicts a multichannel device for manipulating and directing droplets. As shown in FIG. 16, microfluidic channels **1601, 1602, 1603, 1604**, and **1605** contain droplets. In a cell culture assay, the droplets **1606** contain cells and cell medium to ensure the health and proliferation of the cells. Microfluidic channel **1600** contain cell medium. To add cell medium to the droplets, microfluidic channel **1607** aligns with microfluidic channel **1600** for a span of time to create a droplet of cell medium. The cell medium droplet merges with a cell droplet **1606,** as discussed above. Microfluidic channel **1607** aligns with microfluidic channel **1609** to allow the droplets containing cells and cell medium to flow into microfluidic channel **1609.** Droplets and/or fluids in microfluidic channel **1609** can be flowed channel plate **1660**. Channel plate **1660** can be moved to align with microfluidic channel **1609,** or microfluidic channel **1609** can be moved to align with channel plate **1670.** It should be appreciated that waste chamber **1611** may align with microfluidic channels **1601, 1602, 1603, 1604,** and **1605** to remove droplets containing non-viable cells. It should also be appreciated that chamber **1617** may align with microfluidic channel **1609** to collect the droplets containing cells for detection, assay, or incubation. Droplets that are not diverted flow from microfluidic channel **1609** into microfluidic channel **1619** when microfluidic channel **1615** is positioned therebetween. It should also be appreciated that chambers **1623** and **1625** can align with microfluidic channel **1619** to divert droplets into chambers **1623** and **1625.** It should be appreciated that chambers **1623** and **1625** may be to collect droplets containing non-viable cells or to incubate the cell containing droplets. Channel plate **1680** containing can align with microfluidic channel **1621** to allow flow of fluids and/or droplets therebetween.

In an alternative embodiment, droplets containing cells may be merged or combined with compounds for investigation of reactivity and efficacy. For example, FIG. 16 depicts a multichannel device for manipulating and directing droplets. As shown in FIG. 16, microfluidic channels **1601, 1602, 1603, 1604,** and **1605** contain droplets. In a cell investigation assay, the droplets **1606** contain cells and cell medium to ensure the health and proliferation of the cells. Microfluidic channel **1600** contains a fluid comprising a test compound. It should be appreciated that any test compound may be used in the assay. To add the target compound to the droplets, microfluidic channel **1607** aligns with microfluidic channel **1600** for a span of time to create a droplet containing the target compound. The target compound droplet merges with a cell droplet **1606,** as discussed above. Microfluidic channel **1607** aligns with microfluidic channel **1609** to allow the droplets containing cells and target compound to flow into microfluidic channel **1609**. It should be appreciated that waste chamber **1611** may align with microfluidic channels **1601, 1602, 1603, 1604,** and **1605** to remove droplets containing non-viable cells. It should also be appreciated that chamber **1617** may align with microfluidic channel **1609** to collect the droplets containing cells for detection, assay, or incubation. Droplets that are not diverted flow from microfluidic channel **1609** into microfluidic channel **1619** when microfluidic channel **1615** is positioned therebetween. It should also be appreciated that chambers **1623** and **1625** can align with microfluidic channel **1619** to divert droplets into chambers **1623** and **1625**. It should be appreciated that chambers **1623** and **1625** may be to collect droplets containing non-viable cells or to incubate the cell containing droplets. In the other preferred embodiments, substrates **1660, 1670,** and/or **1680** can align to receive fluids and/or droplets.

A multichannel system is depicted in FIG. 18. In regards to FIG. 18, the microfluidic channels and chambers may be aligned by sliding the microfluidic channels proximately to one another. Microfluidic channels may be slid together or independently of one another, as discussed above. Chambers may also be slid in together, or independently. Microfluidic channels that are slid together may be located on the same substrate, or may be located on different substrates. Similar arrangement can be with chambers. As discussed above, alignment may create air gaps (not shown). The liquid in the channels or chambers bridge the air gap to allow fluid and/or droplets to flow from a microfluidic channel to another microfluidic channel, or from a microfluidic channel to a chamber. As shown in FIG. 18, multiple microfluidic channels **1810, 1812, 1814, 1815, 1817, 1819,** and **1821** contain droplets, fluids, or fluids containing target compounds. A detector **1822** is positioned proximate to microfluidic channel **1821**. A detector can be positioned proximate to any microfluidic channel in the multichannel device. In an aspect of the invention, cells may be combined with target compounds to test or analyze the effects on cells. As depicted in FIG. 18, chamber **1828** receives a droplet from any of microfluidic channels **1810, 1812, 1814, 1815, 1817, 1819,** or **1821**. Chamber **1828** can be for waste or for incubating the droplet for a span of time. Similarly, chamber **1832** can receive a droplet from any of microfluidic channels **1810, 1812, 1814, 1815, 1817, 1819,** or **1821**. Chamber **1832** can be for waste, or to incubate the cells for any span of time. A heating element or heating source, not shown, may be proximately located to either chambers **1828** and **1832**. As shown in FIG. 18, microfluidic channel **1830** received a droplet **1829** from any of microfluidic channels **1810, 1812, 1814, 1815, 1817, 1819,** or **1821**. Droplet **1829** passes through microfluidic channel **1830** and is then passed to a channel within channel plate **1844**. Channel plate **1844** contains branched channels **1845**. Droplets entering channel plate **1844** can be distributed through channel plates **1851** and **1861**. It should be appreciated that the channel plates can be aligned to for varying intervals of time.

As shown in FIG. 18, microfluidic channels **1824** and **1826** can receive fluids and/or droplets from any of microfluidic channels **1810, 1812, 1814, 1815, 1817, 1819,** or **1821**. For example, microfluidic channel **1824** can align with microfluidic channel **1812** to receive a droplet. Microfluidic channel **1824** can then align with microfluidic channel **1810** to form a droplet from the fluid in microfluidic channel **1810**. The fluid can contain reactants, cell medium, or target compounds. In microfluidic channel **1824**, droplets from microfluidic channels **1810** and **1812** can coalesce. Similarly, microfluidic channel **1826** can receive droplets and fluids from any of microfluidic channels **1810, 1812, 1814, 1815**, **1817**, **1819,** or **1821**. For example, microfluidic channel **1826** can receive droplets from microfluidic channel **1814** and can form droplets from the fluid contained in microfluidic channel **1815**. The droplets from microfluidic channels **1814** and **1815** can be coalesced by techniques and methods discussed above. By way of example, microfluidic channel may contain target compounds that are combined with droplets in microfluidic channel **1812**. Microfluidic channel **1840** can align with microfluidic channels **1824** and **1826** to receive the droplets contained within microfluidic channels **1824** and **1826**. Microfluidic channel **1850** can align with microfluidic channels **1840** or **1848**. Microfluidic channel **1848** can contain fluid, which contains reactants, cell medium, or target compounds. By aligning with microfluidic channel **1848,** microfluidic channel **1850** can create droplets from the fluid contained within microfluidic channel **1848**. Droplets from microfluidic channel **1840** and **1848** can be coalesced in microfluidic channel **1850** by the methods and techniques discussed above. Droplets in microfluidic channel **1850** can be diverted into microfluidic channel **1873** by aligning microfluidic channels **1850** and **1873**. Droplets in microfluidic channel **1850** can be diverted to chamber **1872,** which can be an incubation chamber. Droplets in microfluidic channel **1873** are diverted into circular microfluidic channel **1880**. Droplets may circulate via a roter, not shown. Droplets may exit circular microfluidic channel **1880** via microfluidic channel **1882**. Channel plate **1887** may align with microfluidic channel **1882**. Furthermore, channel plate **1870** may align with channel plate **1887**.

For example, in a preferred embodiment, cells are encased in the droplets contained in microfluidic channels **1819** and **1821**. Microfluidic channel **1830** aligns with microfluidic channel **1819** to receive droplets. The droplets are flowed channel plate **1844** to distribute the droplets. Droplets, and thereby cells, are flowed into channel plates **1865** and **1851**. Channel plate **1865** aligns with channel plate **1861** and then channel plate **1865**. Channel plates **1861** and **1865** can contain fluids that contain nutrients, nucleic acids, molecules, compounds, etc.

For example, in another preferred embodiment, droplets in microfluidic channel **1812** contain cells, and microfluidic channel **1810** contains a fluid containing a testing compound, (e.g., a drug molecule). Microfluidic channel **1824** aligns with microfluidic channel **1812** to receive a droplet and aligns with microfluidic channel **1810** to flow a small volume of the fluid in microfluidic channel **1824**. The droplet and small volume are positioned closely to cause passive merging, as discussed above. Microfluidic channel **1848** aligns with microfluidic channel **1824** to receive a coalesced droplet and aligns with microfluidic channel **1826** to receive a small volume of the fluid contained in microfluidic channel **1826**. The fluid in microfluidic channel may be cell medium, reactants, nutrients, buffer, etc. Microfluidic channel **1850** aligns with microfluidic channel **1848** to direct the droplets to circular microfluidic channel **1880** via microfluidic channel **1873**. The droplets are circulated for a period of time and then diverted to microfluidic channel **1882**. Channel plate **1887** can align with microfluidic channel **1882** to receive droplets. The droplets can be further distributed into channel plate **1870**. Droplets are then flowed into a chamber (not shown) for detection.

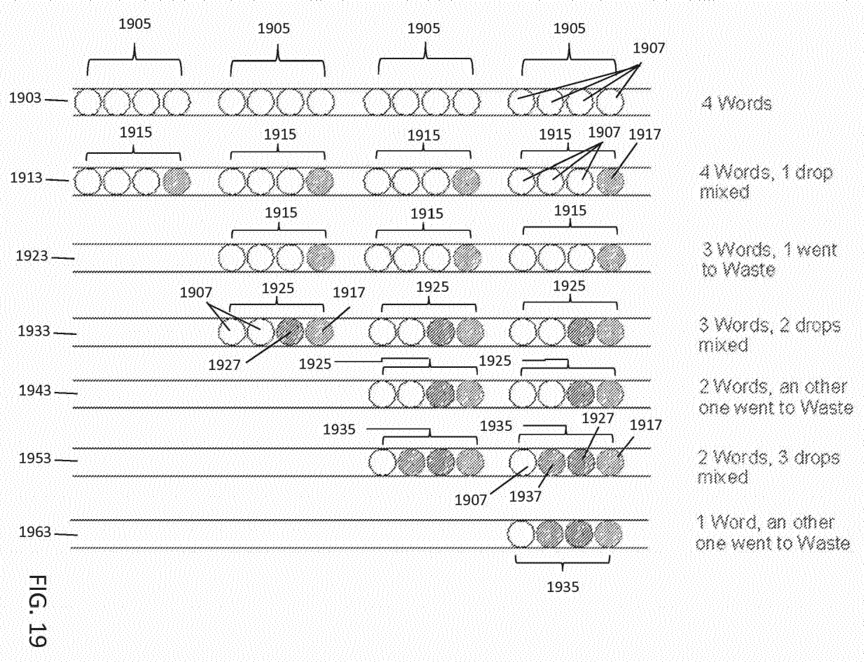

In an aspect of the invention, combinatorial methods may be employed in assays using the device of the invention. Any combinatorial approach or strategy known in the art may be used with systems of the invention. FIG. 19 provides an exemplary embodiment, showing that droplets may be organized into groups, called Words. A Word, as used herein, is a plurality of n droplets. Each droplet is a letter in the Word. For example, a Word comprising five droplets would correlate to a Word of five letters. For example, if a Word contains three droplets, and each droplet represents a letter, then the Word may be arranged as aaa, bbb, ccc, abc, abb, acc, acb, etc. In FIG. 19, four Words **1905** are contained within a microfluidic channel **1903**. Each Word **1905** comprises four droplets **1907**, or four letters **1907**. Each letter, or droplet in Word **1903** is the same and may, for example, be represented as aaaa. As shown in FIG. 19, four Words **1915** comprise four droplets, or four letters, of three droplets **1907** and one droplet **1917** (represented, for example, as aaab). Droplet **1917** is formed by mixing a droplet **1907** with additional materials, reactants, etc. Mixing or coalescing of droplets is described above. As shown in FIG. 19, microfluidic channel **1923** contains three Words 1915, as one Word **1915** was directed to waste, methods of which are described above. As shown in FIG. 19, three Words **1925** are contained in microfluidic channel **1933**. Each Word **1925** contains two droplets of **1907**, one droplet **1917**, and one droplet **1927** (represented, for example, as aacb). Droplet 1927 can be formed by mixing droplet **1907** with additional materials, reactants, etc. Mixing or coalescing of droplets is described above. In microfluidic channel **1943**, contains two Words **1925**, where one Word **1925** was directed to waste, methods of which are described above. Microfluidic channel 1953 contains two Words **1935**, in which each Word **1935** contains one droplet **1907**, one droplet **1917**, one droplet **1927**, and one droplet **1937** (represented, for example, as adcb). Droplet **1937** can be formed by mixing droplet **1907** with additional materials, reactants, etc. Microfluidic channel **1963** contains one Word **1935**.

FIG. 19 depicts a work flow process in which Words are processed through a microfluidic device of the invention. Microfluidic channel **1903** contains Words **1905**, in which each Word **1905** contains similar droplets **1907**. The droplets **1907** may contain any components or materials described herein. For example, each droplet **1907** may contain a cell. The Words **1905** are flowed into microfluidic channel **1913**, and a droplet **1907** is coalesced with another droplet to form droplet **1917**. For example, a cell in a droplet **1907** may be merged or mixed with a droplet containing a target compound). The Words **1915** are then flowed into microfluidic channel **1923**, however, one Word was diverted to waste, thereby only three Words were flowed into microfluidic channel **1923**. As described above, droplets, or Words may be diverted to waste by aligning microfluidic channel **1913** with a waste chamber. For example, a detector may have detected a deficiency, error, or abnormality, causing the diversion to waste. Words **1915** are flowed into microfluidic channel **1933** and a droplet **1907** is coalesced with another droplet to form droplet **1927**, forming Word **1925**. For example, a droplet **1907** may be merged with a droplet containing the same or different target compound, a reactant, or any other species. Words **1925** are flowed into microfluidic channel 1953, however, one Word **1925** was diverted to waste, thereby only two Words were flowed into microfluidic channel **1943**. As described above, an abnormality may have been detected and the Word was diverted into a waste chamber. Words **1925** are flowed into microfluidic channel **1953** and a droplet **1907** is coalesced with another droplet to form droplet **1937**, forming Word **1935**. The droplet merged with droplet **1907** may contain the same or different target compound, reactants, or any other material. Words **1935** are then flowed into microfluidic channel **1963**, however, one Word was diverted to waste, thereby only one Word **1935** was flowed into microfluidic channel **1963**. As stated above, the diversion could have been based upon the detection of an abnormality. In an aspect of the invention a droplet, or letter, may contain an identifying tag to identify the Word.

In an exemplary embodiment of the invention, iPS cells are transformed from somatic cells, cultured, and used in expression profiling employing devices of the present invention. Any method known in the art may be employed to transform somatic cells into iPS cells. In certain embodiments, an indirect route that involves dedifferentiation and then redifferentiation of the somatic cells is employed. Such a route involves reprogramming a variety of somatic cell types from different lineages to produce a dedifferentiated embryonic stem cell state. Indirect routes include somatic cell nuclear transfer, cell fusion, or creation of induced pluripotent stem cells by introduction of genes such as Oct4. The dedifferentiated cells are then redifferentiated to target cells along respective mesodermal, endodermal, or ectodermal lineages. Further description of such methods are found for example in Isacson et al. (U.S. patent application number 2010/0021437), Yamanaka et al. (U.S. patent application number 2009/0047263), Sakurada et al. (U.S. patent application number 2009/0191159), Yamanaka et al. (U.S. patent application number 2009/0227032), Sakurada et al. (U.S. patent application number 2009/0304646), Sakurada et al. (U.S. patent application number 2010/0105100), Takahashi et al. (U.S. patent application number 2010/0105137), Sakurada et al. (U.S. patent application number 2010/0120069), Sakurada et al. (U.S. patent application number 2010/0267135), Hochedlinger et al. (U.S. patent application number 2010/0062534), and Hochedlinger et al. (U.S. patent application number 2010/0184051), the content of each of which is incorporated by reference herein in its entirety. Methods for preparing induced pluripotent stem cells by using a nuclear reprogramming factor are described in International publication number WO 2005/80598, the content of which is incorporated by reference herein in its entirety.

Employing the methods discussed herein, iPS cells are encased in droplets containing cell medium and related growth factors. The iPS cell droplets are cultured by techniques and methods discussed above, or known in the art. iPS cell droplets can be merged with other materials and reactants, including maintenance medium, transfection reagents, etc. during the incubation process.

Overall, the process of inducing cells to become multipotent or pluripotent is based on forcing the expression of polypeptides, particularly proteins that play a role in maintaining or regulating self-renewal and/or pluripotency of ES cells. Examples of such proteins are the Oct3/4, Sox2, Klf4, and c-Myc transcription factors, all of which are highly expressed in ES cells. Forced expression may include introducing expression vectors encoding polypeptides of interest into cells (Hochedlinger et al., U.S. patent application number 2010/0062534), transduction of cells with recombinant viruses, introducing exogenous purified polypeptides of interest into cells, contacting cells with a non-naturally occurring reagent that induces expression of an endogenous gene encoding a polypeptide of interest (e.g., Oct3/4, Sox2, Klf4, or c-Myc), or any other biological, chemical, or physical means to induce expression of a gene encoding a polypeptide of interest (e.g., an endogenous gene Oct3/4, Sox2, Klf4, or c-Myc). Some basic steps to induce the cells are shown in Sakurada et al. (U.S. patent application number 2009/0191159). These steps may involve: collection of cells from a donor, e.g., a human donor, or a third party; induction of the cells, e.g., by forcing expression of polypeptides such as Oct3/4, Sox2, Klf4, and c-Myc (110); identifying multipotent or pluripotent stem cells; isolating colonies; and optionally, storing the cells. Interspersed between all of these steps are steps to maintain the cells, including culturing or expanding the cells. In addition, storage of the cells can occur after many steps in the process. Cells may later be used in many contexts, such as therapeutics or other uses.

iPS cell droplets may be used in expression profiling where target compounds are introduced into the droplets by methods disclosed herein. In order to use expression analysis for disorder diagnosis, a threshold of expression is established. The threshold may be established by reference to literature or by using a reference sample from a subject known not to be afflicted with the disorder. The expression may be over-expression compared to the reference (i.e., an amount greater than the reference) or under-expression compared to the reference (i.e., an amount less than the reference). In, expression profiling, the iPS cell droplets are merged with droplets containing target compounds and allowed to further incubate, which may involve splitting of droplets, i.e. splitting of cell cluster, and merging of droplets, i.e. introduction of freshcell medium. Methods of the invention may be used to detect any disorder or compound effect. The iPS cell droplets may be flowed passed a detector to screen for abnormalities, or diverted to a collection chamber for analysis.

While preferred embodiments of the present invention have been shown and described herein, it will be obvious to those skilled in the art that such embodiments are provided by way of example only. Numerous variations, changes, and substitutions will now occur to those skilled in the art without departing from the invention, It should be understood that various alternatives to the embodiments of the invention described herein may be employed in practicing the invention. It is intended that the following claims define the scope of the invention and that methods and structures within the scope of these claims and their equivalents be covered thereby.

Incorporation by Reference

References and citations to other documents, such as patents, patent applications, patent publications, journals, books, papers, web contents, have been made throughout this disclosure. All such documents are hereby incorporated herein by reference in their entirety for all purposes.

Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.

**Numbered clauses relating to the invention**

1. An apparatus for fluid handling, the apparatus comprising:

    a body with a top surface and a bottom surface; and
    a well within the body, wherein the well comprises an opening at the top surface and an opening at the bottom surface; and wherein opening at the bottom surface is configured such that a fluid is retained in the well when the body is oriented top-side up.

2. The apparatus of clause 1, wherein the opening at the bottom surface is dimensioned such that the fluid is retained in the well by surface tension when the body is oriented top-side up.

3. The apparatus of clause 1, further comprising a hydrophobic surface within at least a portion of the well.

4. The apparatus of clause 3, wherein the hydrophobic surface is adjacent the opening at the bottom surface.

5. The apparatus of clause 4, wherein the hydrophobic surface extends from within the well over at least a portion of the bottom surface.

6. The apparatus of clause 1, further comprising a plurality of wells.

7. The apparatus of clause 1, wherein the opening at the bottom surface is less than 2 mm across in all directions.

8. The apparatus according to clause 1, wherein the opening at the top surface consists of a single circular opening.

9. The apparatus according to clause 1, wherein the opening at the bottom surface comprises a circular opening with a diameter less than or equal to 2 mm.

10. The apparatus according to clause 1, wherein the opening at the top surface is larger than the opening at the bottom surface.

11. The apparatus according to clause 1, wherein the well defines a cylindrical inner wall.

12. The apparatus according to clause 1, wherein at least a portion of the interior of the well is tapered or conical.

13. The apparatus according to clause 8, wherein a portion of the fluid receptacle has a wider diameter than the opening at the bottom surface.

14. The apparatus according to clause 1, wherein the well comprises a second opening at the bottom surface.

15. The apparatus according to clause 1, wherein a portion of the well has a narrower diameter than the opening at the bottom surface.

16. A method for handling fluid, the method comprising:

    providing an apparatus for fluid handling, the apparatus comprising:

        a body with a top surface and a bottom surface, and
        a well within the body, wherein the well has an opening at the top surface and an opening at the bottom surface;

    loading a fluid into the well, wherein the fluid is retained in the well by surface tension when the body is oriented top-side up;
    bringing a device comprising a channel into contact with the fluid at the opening at the bottom surface, thereby causing at least a portion of the fluid to flow into the channel.

17. The method of clause 16, wherein the fluid is an oil comprising aqueous droplets.

18. The method of clause 17, wherein the droplets comprise cells.

19. The method of clause 16, further comprising a second fluid retained within the channel.

20. The method of clause 14, further comprising bringing the device into contact with the fluid to cause only a single droplet to flow into each channel.

21. The method of clause 16, wherein gravity causes the flow of fluid from the well to the channel when the well and the channel are aligned.

22. The method according to clause 16, wherein the well opening at the top surface comprises a single circular opening.

23. The method according to clause 16, wherein the well opening at the bottom surface comprises a single circular opening.

24. The method according to clause 16, wherein the well opening at the bottom surface has a smaller diameter than the opening at the top surface.

25. The method according to clause 16, wherein the well opening in the top surface has a larger diameter than the well opening in the bottom surface.

26. The method according to clause 16, wherein the interior walls of the well are linear.

27. The method according to clause 16, wherein the interior walls of the fluid receptacle are non- linear.

28. The method according to clause 16, wherein the well opening at the bottom surface comprises two openings.

29. The method according to clause 13 wherein the wells are associated with a barcode.

30. The method according to clause 13, wherein the walls of the wells are hydrophobic.


**Claims**

1. An apparatus for fluid handling, the apparatus comprising:

   a body with a top surface and a bottom surface; and
   a well within the body, wherein the well comprises an opening at the top surface and an opening at the bottom surface; and wherein opening at the bottom surface is configured such that a fluid is retained in the well when the body is oriented top-side up.

2. The apparatus of claim 1, wherein the opening at the bottom surface is dimensioned such that the fluid is retained in the well by surface tension when the body is oriented top-side up.

3. The apparatus of claim 1, further comprising a hydrophobic surface within at least a portion of the well.

4. The apparatus of claim 3, wherein the hydrophobic surface is adjacent the opening at the bottom surface.

5. The apparatus of claim 4, wherein the hydrophobic surface extends from within the well over at least a portion of the bottom surface.

6. The apparatus of claim 1, further comprising a plurality of wells.

7. The apparatus of claim 1, wherein the opening at the bottom surface is less than 2 mm across in all directions.

8. The apparatus according to claim 1, wherein the opening at the top surface consists of a single circular opening.

9. The apparatus according to claim 1, wherein the opening at the bottom surface comprises a circular opening with a diameter less than or equal to 2 mm.

10. The apparatus according to claim 1, wherein the opening at the top surface is larger than the opening at the bottom surface.

11. The apparatus according to claim 1, wherein the well defines a cylindrical inner wall.

12. The apparatus according to claim 1, wherein at least a portion of the interior of the well is tapered or conical.

13. The apparatus according to claim 8, wherein a portion of the fluid receptacle has a wider diameter than the opening at the bottom surface.

14. The apparatus according to claim 1, wherein the well comprises a second opening at the bottom surface.

15. The apparatus according to claim 1, wherein a portion of the well has a narrower diameter than the opening at the bottom surface.

FIG. 1

FIG. 2A

204

205

202

206

FIG. 2B

213

210

212

209

FIG. 2C

223

220

221

219

FIG. 2D

231

230

235

233

FIG. 3

FIG. 4A

FIG. 4B

FIG. 5A

FIG. 5B

FIG. 5C

501

515

504

FIG. 5D

590

506

601    603    605

630    632    634    606

602    603

651    650

609    611

FIG. 6A

FIG. 6B

FIG. 6C

FIG. 7A

FIG. 7B

FIG. 7C

FIG. 8A

810  811  812  813  814  815  816  817

801  802  803  804  805  806  807  808

FIG. 8B

810  811  812  813  814  815  816  817

801  802  803  804  805  806  807  808

FIG. 8C

810  811  812  813  814  815  816  817

801  802  803  804  805  806  807  808

FIG. 9A

FIG. 9B

FIG. 10A

FIG. 10B

FIG. 10C

FIG. 10D

FIG. 10E

FIG. 11A

FIG. 11B

FIG. 11C

FIG. 12A

1220 1221 1222 1223

1202

1203

1213

1210

1212

1211

1204

1216 1215

1218 1219 1206

FIG. 12B

1220 1221 1222 1223

1202

1203

1205

1210

1213

1211

1212

1204

1216 1215

1218 1219

1206

FIG. 12C

FIG. 12D

FIG. 13A

FIG. 13B

1401   1403

1405

FIG. 14A

1401   1403

1405

FIG. 14B

FIG. 14C

FIG. 14D

FIG. 14E

FIG. 15

FIG. 16

FIG. 17A

1707

1705

1709

FIG. 17B

1761

1765

1764

1762

FIG. 18

FIG. 19

FIG. 20

FIG. 21

FIG. 22

FIG. 23A

FIG. 23B

Europäisches
Patentamt

European
Patent Office

Office européen
des brevets

# EUROPEAN SEARCH REPORT

Application Number

EP 19 15 7685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 051 259 A1 (MASSACHUSETTS INST TECHNOLOGY [US]) 15 November 2000 (2000-11-15) * paragraphs [0011], [0019] - [0031]; figures 1,5,9-10 * ----- | 1-15 | INV. B01L3/00 C12N5/00 B01F13/00 B01L7/00 B01F3/08 C12M1/32 C12M3/06 C12Q1/18 G01N33/50 |
| X | US 2002/001546 A1 (HUNTER IAN [US] ET AL) 3 January 2002 (2002-01-03) * paragraphs [0049] - [0052], [0057], [0095], [0119] - [0123]; figures 3, 6, 15-19 * ----- | 1-15 | |
| X | US 2003/124599 A1 (CHEN SHIPING [US] ET AL) 3 July 2003 (2003-07-03) * paragraphs [0192], [0217] - [0237], [0257] - [0285]; figures 11,17,22-31, * ----- | 1-15 | |
| X | US 2002/151040 A1 (O' KEEFE MATTHEW [US] ET AL) 17 October 2002 (2002-10-17) * paragraphs [0064] - [0084], [0146], [0167]; figures 1-5 * ----- | 1-15 | TECHNICAL FIELDS SEARCHED (IPC) |
| X | WO 2013/138767 A1 (LIFE TECHNOLOGIES CORP [US]) 19 September 2013 (2013-09-19) * paragraphs [0051] - [0056]; figures 7-16, 21, 22 * ----- | 1-15 | B01L C12N |

The present search report has been drawn up for all claims

| Place of search | Date of completion of the search | Examiner |
|---|---|---|
| The Hague | 3 June 2019 | Viskanic, Martino |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 15 7685

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2019

| Patent document cited in search report | | Publication date | Patent family member(s) | | Publication date |
|---|---|---|---|---|---|
| EP 1051259 | A1 | 15-11-2000 | AT | 322341 T | 15-04-2006 |
| | | | AT | 477850 T | 15-09-2010 |
| | | | AU | 2102699 A | 26-07-1999 |
| | | | CA | 2316912 A1 | 15-07-1999 |
| | | | DE | 69930726 T2 | 25-01-2007 |
| | | | EP | 1051259 A1 | 15-11-2000 |
| | | | EP | 1714699 A1 | 25-10-2006 |
| | | | EP | 2286918 A1 | 23-02-2011 |
| | | | ES | 2350702 T3 | 26-01-2011 |
| | | | JP | 4271371 B2 | 03-06-2009 |
| | | | JP | 4448166 B2 | 07-04-2010 |
| | | | JP | 4668334 B2 | 13-04-2011 |
| | | | JP | 2002500373 A | 08-01-2002 |
| | | | JP | 2008139319 A | 19-06-2008 |
| | | | JP | 2009244271 A | 22-10-2009 |
| | | | US | 6387331 B1 | 14-05-2002 |
| | | | US | 6743633 B1 | 01-06-2004 |
| | | | US | 2004171166 A1 | 02-09-2004 |
| | | | US | 2011065590 A1 | 17-03-2011 |
| | | | US | 2015126412 A1 | 07-05-2015 |
| | | | US | 2015298089 A1 | 22-10-2015 |
| | | | WO | 9934920 A1 | 15-07-1999 |
| US 2002001546 | A1 | 03-01-2002 | CA | 2446157 A1 | 14-11-2002 |
| | | | CA | 2665706 A1 | 14-11-2002 |
| | | | EP | 1385628 A2 | 04-02-2004 |
| | | | JP | 4607202 B2 | 05-01-2011 |
| | | | JP | 4741042 B2 | 03-08-2011 |
| | | | JP | 5103536 B2 | 19-12-2012 |
| | | | JP | 2005507492 A | 17-03-2005 |
| | | | JP | 2008268202 A | 06-11-2008 |
| | | | JP | 2008292478 A | 04-12-2008 |
| | | | JP | 2009096992 A | 07-05-2009 |
| | | | JP | 2011095271 A | 12-05-2011 |
| | | | US | 2002001546 A1 | 03-01-2002 |
| | | | US | 2004191924 A1 | 30-09-2004 |
| | | | US | 2005079105 A1 | 14-04-2005 |
| | | | US | 2009258797 A1 | 15-10-2009 |
| | | | US | 2011319300 A1 | 29-12-2011 |
| | | | WO | 02089982 A2 | 14-11-2002 |
| US 2003124599 | A1 | 03-07-2003 | US | 2003124599 A1 | 03-07-2003 |
| | | | WO | 03042697 A1 | 22-05-2003 |
| US 2002151040 | A1 | 17-10-2002 | US | 2002151040 A1 | 17-10-2002 |
| | | | US | 2005148066 A1 | 07-07-2005 |

EPO FORM P0459

For more details about this annex : see Official Journal of the European Patent Office, No. 12/82

page 1 of 2

**EP 3 539 662 A1**

## ANNEX TO THE EUROPEAN SEARCH REPORT
## ON EUROPEAN PATENT APPLICATION NO.

EP 19 15 7685

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2019

| Patent document cited in search report | Publication date | Patent family member(s) | Publication date |
|---|---|---|---|
| | | US 2010075330 A1 | 25-03-2010 |
| | | US 2012077194 A1 | 29-03-2012 |
| | | US 2015087550 A1 | 26-03-2015 |
| | | US 2017101667 A1 | 13-04-2017 |
| | | US 2018245133 A1 | 30-08-2018 |
| WO 2013138767 A1 | 19-09-2013 | AU 2013231910 A1 | 30-10-2014 |
| | | CN 104302400 A | 21-01-2015 |
| | | CN 104411408 A | 11-03-2015 |
| | | EP 2825310 A2 | 21-01-2015 |
| | | EP 2825314 A1 | 21-01-2015 |
| | | JP 2015511015 A | 13-04-2015 |
| | | JP 2015516802 A | 18-06-2015 |
| | | KR 20140140080 A | 08-12-2014 |
| | | KR 20150003738 A | 09-01-2015 |
| | | RU 2014140837 A | 10-05-2016 |
| | | RU 2014141637 A | 10-05-2016 |
| | | SG 11201405785W A | 27-11-2014 |
| | | SG 11201406039W A | 27-11-2014 |
| | | US 2015044686 A1 | 12-02-2015 |
| | | US 2015080247 A1 | 19-03-2015 |
| | | WO 2013138706 A2 | 19-09-2013 |
| | | WO 2013138767 A1 | 19-09-2013 |

EPO FORM P0459

For more details about this annex : see Official Journal of the European Patent Office, No. 12/82

page 2 of 2

65

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61993119 B
- US 62115872 B
- US 62115877 B
- US 5523162 A
- US 6210986 B
- US 5885470 A
- WO 2002063288 A
- US 3397278 A
- US 3506424 A
- US 4038139 A
- US 7258998 B
- US 13497707 B
- US 8338177 B
- US 13695002 B
- US 20100294048 A, McGuire
- US 20080014589 A, Link
- US 20080003142 A
- US 20100137163 A
- US 7708949 B, Stone
- US 20100172803 A
- US 7041481 B, Anderson
- US 20050172476 A
- WO 2004002627 A

- US 20140017150 A
- US 02422804 A
- WO 2013014215 A
- US 20110244455 A
- WO RE41780 A
- EP 2047910 A
- US 6143496 A, Brown
- US 6391559 B
- US 6440706 B, Vogelstein
- US 6753147 B
- US 7824889 B
- US 20100021437 A, Isacson
- US 20090047263 A, Yamanaka
- US 20090191159 A, Sakurada
- US 20090227032 A, Yamanaka
- US 20090304646 A, Sakurada
- US 20100105100 A, Sakurada
- US 20100105137 A, Takahashi
- US 20100120069 A, Sakurada
- US 20100267135 A, Sakurada
- US 20100062534 A, Hochedlinger
- US 20100184051 A, Hochedlinger
- WO 200580598 A

**Non-patent literature cited in the description**

- **M. STJEMSTROM ; J. ROERAADE.** Method for Fabrication of Microfluidic Systems in Glass. *J. Micromechanics and Microenginneering,* 1998, vol. 8, 33-38
- **A. BERTHOLD.** Glass-to-glass anodic bonding with standard ICAechnology thin films as intermediate layers. *Sensors & Actuators A,* 2000, vol. 82, 224-228
- **H.Y. WANG et al.** Low temperature bonding for microfabrication of chemical analysis systems. *Sensors & Act. B,* 1997, vol. 45, 199-207
- **DJ. HARRISON.** Capillary electrophoresis and sample injection systems integrated on a planar glass chip. *Analytical Chemistry,* 01 September 1992, vol. 64, 1926

- **XU et al.** Droplet Coalescence in Microfluidic Systems. *Micro and Nanosystems,* 2011, vol. 3, 131-136
- **GU et al.** *Int J Mol Sci.,* 2011, vol. 12 (4), 2572-2597
- **LINK et al.** *Phys. Rev. Lett.,* 2004, vol. 92, 4
- **NIE et al.** *Anal. Chem.,* 2010, vol. 82, 7852-7856
- **MANIATIS et al.** Molecular Cloning: A Laboratory Manual. Cold Spring Harbor, 1982, 280-281
- **TSAI et al.** Isolation of human multipotent mesenchymal stem cells from second-trimester amniotic fluid using a novel two-stage culture protocol. *Hum. Reprod.,* 2004, vol. 19 (6), 1450-1456